# EUROPEAN PATENT APPLICATION

(11) **EP 3 900 676 A1**
(43) Date of publication of application: **27.10.2021**
(21) Application number: 21170306.1
(22) Date of filing: 23.04.2021
(51) Int. Cl.: A61F 2/00, A61B 17/00

(54) **KITS FOR SURGICAL REPAIR OF SOFT TISSUE DEFECTS AND COMPONENTS, PACKAGING, AND METHODS OF USE THEREOF**

(30) Priority: 23.04.2020 EP 20315214
(71) Applicant: Sofradim Production, 01600 Trévoux (FR)
(72) Inventor: Bailly, Pierre, 69300 Caluire et Cuire (FR); JOUVRAY, Guy, 38119 Pierre Chatel (FR); BRUNE, Thierry, 69640 Jamioux (FR)
(74) Representative: Maschio, Antonio

(57) **Abstract**

The present disclosure describes kits for surgical repair of soft tissue defects, including hernias.

The kits include any combination of components selected from an implantable sheet, a central tie, a delivery device, an insertion member, and/or a rolling device. Packaging for the kits and/or components and methods of using the kits and/or components are also provided.

## Description

### Cross-Reference to Related Applications

This application claims benefit of and priority to EP20315213.7 filed 23 April 2020 and EP 20315214.5 filed 23 April 2020, the disclosure of the above-identified applications are hereby incorporated by reference in their entirety.

### Technical Field

The present disclosure describes kits for surgical repair of soft tissue defects, including hernias, and particularly to the components, packaging, and methods of use of such kits.

### Background

Various prosthetic repair materials are employed by surgeons for soft tissue repair including the repair of anatomical defects such as tissue and muscle hernias. For example, a ventral hernia in the abdominal wall is commonly repaired using an implantable sheet of biocompatible fabric, such as a knitted mesh (PARIETEX™, VERSATEX™, and the like) or a composite fabric that includes a mesh and an adhesion resistant barrier (SYMBOTEX™, PARIETENE™, and the like). The fabric is typically sutured, stapled, tacked, glued, or otherwise provisionally anchored in place over, under or within the defect. Tissue integration with the fabric, such as tissue ingrowth into and/or along the mesh fabric, eventually completes the repair. An implantable sheet of adhesion resistant barrier material, if provided alone or in combination with a fabric, prevents the growth of fibrous adhesions between the bowel (and other organs located in the abdominal cavity) and the sheet or fabric, specifically when the sheet is implanted inside the abdominal cavity (i.e. under the defect).

Various surgical techniques may be employed for soft tissue repair, including open or laparoscopic procedures. In addition, these surgical techniques may be performed directly by surgeon or with the assistance of a surgical robot. During a laparoscopic procedure, the prosthetic fabric may be routed, directly by the surgeon or with the assistance of a surgical robot, to the surgical site through a slender laparoscopic or robotic cannula. The fabric is typically collapsed, such as by rolling or folding, into a reduced configuration to facilitate its passage through the narrow cannula. Certain repairs, such as laparoscopic repair of ventral hernias, may require large sheets of prosthetic fabric that may be difficult to deliver laparoscopically, as well as difficult to properly deploy, orientate, position, or fixate following delivery.

Preparation and/or delivery of the prosthetic fabric can critically impact later steps of the surgical procedure. In laparoscopic procedures, prosthetic fabrics are typically prepared and delivered into a small operating space. This can make the deployment, orientation, positioning, and/or fixating of the fabric more difficult and more time consuming. It can also require the surgeon to dedicate one hand to simply trying to maintain the fabric in a certain position while the surgeon's second hand is trying to fixate the fabric in the tissue. This can be particularly challenging since the edges of the fabrics tend to bend or fold inside the small workspace. Mispositioning of the fixated prosthetic fabric can potentially lead to hernia recurrence.

It is an object of the present disclosure to provide kits and/or components of a kit which are designed to make preparation, insertion, deployment, orientation, positioning, and/or fixation of an implantable sheet easier, more intuitive and less time-consuming thereby rendering the surgical procedure more efficient and more effective.

It is another object of the present disclosure to provide kits and/or components of a kit which are designed to be prepared or delivered in a manner which allows a surgeon, directly or with the assistance of a surgical robot, to dedicate multiple hands to handle, deploy, orientate, position, and/or fixate the implantable sheet, during a standard laparoscopic or a robotically assisted ventral hernia repair.

### Summary

Surgical kits for soft tissue defect repair are described herein. The surgical kits include a combination of components selected from an implantable sheet, a central tie, a delivery device, a rolling device, and an insertion member. The delivery device may be a one-piece delivery device or a multiple-piece delivery device configured to deliver an implantable sheet into a patient.

In some embodiments, the delivery device may be a one-piece delivery device including a body member extending from a proximal end portion to a distal end portion. The body member includes one or more resilient arms and a central arm positioned between the proximal and distal end portions thereof. At least one of the one or more resilient arms or the central arm are affixed to both the proximal and distal end portions of the body member to form the one-piece delivery device. In some embodiments, the body member includes at least a pair of resilient arms.

The distal end portion of the body member includes a slot defined therein. The slot separates the distal end portion of the body member into an upper and lower jaw member. The slot is configured to secure a distal end portion of an implantable sheet between the upper and lower jaw members.

The body member, including the one or more resilient arms, is configured to transition between an expanded or open configuration and a restrained or closed configuration. In the expanded configuration the one or more resilient arms are spaced farthest from the central arm or farthest apart from each other. In the restrained or closed configuration, the one or more resilient arms are generally adjacent the central arm or generally adjacent each other.

In some embodiments, the central arm of the one-piece delivery device is affixed to both the proximal and distal end portions of the body member and the one or more resilient arms are affixed to the distal end portion of the body member or both the distal and proximal end portions of the body member.

In some embodiments, the central arm of the one-piece delivery device is affixed to both the proximal and distal end portions of the body member and the one or more resilient arms are affixed to only the proximal end portion of the body member and free of the distal end portion of the body member.

In some embodiments, the one or more resilient arms of the one-piece delivery device are affixed to both the proximal and distal end portions of the body member and the central arm is affixed to the distal end portion of the body member or both of the proximal and distal end portions of the body member.

In some embodiments, the one-piece delivery device includes at least a pair of resilient arms and a resilient central arm positioned therebetween. The resilient central arm may include a generally S-shaped central portion in the expanded configuration and may be generally adjacent the pair of resilient arms in the restrained configuration.

In some embodiments, the proximal end portion of the body member of the one-piece delivery device may include a proximal tube configured to receive and maintain a proximal end portion of each of the pair of resilient arms therein, while the distal end portion of each of the pair of resilient arms are affixed to the distal end portion of the body member.

In some embodiments, the central arm is affixed to both the proximal and distal end portions of the body member of the one-piece delivery device and includes a central gap splitting the central arm into a first arm part space longitudinally from a second arm part. The first arm part is affixed to the proximal end portion of the body member and the second arm part is affixed to the distal end portion of the body member. The central arm may also include a first and second tie holes wherein the first tie hole is defined through a distal end portion of the first arm part and the second tie hole is defined through a proximal end portion of the second arm part.

In some embodiments, the delivery device may be a one-piece delivery device including a plurality of pairs of resilient arms including at least a first and second pair of resilient arms, the first pair of resilient arms affixed to the proximal end portion of the body member, the second pair of resilient arms affixed to the distal end portion of the body member, and a central arm positioned at least between the first and second pairs of resilient arms, wherein at least one of the first or second pair of resilient arms are configured to transition between an expanded configuration and a restrained configuration. The central arm may further extend from the proximal end portion of the body member to a distal end portion of the first pair of resilient arms, the proximal end portion of the second pair of resilient arms to the distal end portion of the body member, or both.

In some embodiments, the delivery device is a multiple-piece delivery device. The multiple delivery device may include a two-piece delivery device configured to deliver an implantable sheet. The two-piece delivery device includes a first and second body member.

In some embodiments, the first body member may include a central arm and the second body member includes one or more resilient arms. In some embodiments, the first body member may include one or more resilient arms and the second body member includes a central arm.

In some embodiments, the first body member of the two-piece delivery device may include a central arm extending between a first proximal end portion and a first distal end portion. The first proximal end portion may include a tube defining a tube lumen. The first distal end portion may include at least one body locking recess defined therein and a slot defined therein. The second body member of the two-piece delivery device may include a pair of resilient arms extending between a second proximal end portion and a second distal end portion. The second proximal end portion may include a handle received within the tube lumen of the first body member. The second distal end portion of the second body member may include one or more locking members configured to be received within the at least one body locking recess of the first body member thereby locking the first body member to the second body member to form the delivery device.

In some embodiments, the two-piece delivery device may include a first and second body members, wherein the first body member includes the resilient arms and the second body member includes a central arm. Particularly, the first body member includes a pair of resilient arms extending between a first proximal end portion and a first distal end portion, the first proximal end portion including a first tube defining a first tube lumen and the first distal end portion including a second tube defining a second tube lumen. The second tube further includes a body locking recess defined therein. The first and second tubes are aligned along the same longitudinal axis. Particularly, the second body member includes one arm extending between a second proximal end portion and a second distal end portion, the second proximal end portion configured to be received within the first tube lumen of the first body member. The second distal end portion includes a body locking member and a slot distal the locking member, the locking member configured to be received within the body locking recess of the first body member locking the first body member to the second body member. The slot separating the second distal end portion of the second body member into an upper and lower jaw member, the slot configured to secure a distal end portion of an implantable sheet between the upper and lower jaw members.

Surgical kits including at least a one-piece or multiple-piece delivery device as described herein are also provided. The surgical kits may further include at least one implantable sheet, an insertion member, a rolling device, or a central tie as provided herein.

In some embodiments, the surgical kit includes a one-piece or multiple-piece delivery device configured to deliver an implantable sheet as described herein and an insertion member including an elongate body, clamp tube, and a finger clip. The elongate body of the insertion member extends between an insertion end portion including a first socket and a rolling end portion including a second socket. The elongate body of the insertion member defining a first longitudinal axis. Each of the first and second sockets are configured to matingly engage the proximal end portion of the delivery device. The clamp tube has a tubular body extending between a first tube end portion and an opposite second tube end portion. The first tube end portion is configured to attach to and extend away from the rolling end portion of the elongate body. The second tube end portion is free of the elongate body of the insertion member. The second tube end portion is configured to receive at least the proximal end portion of the delivery device therein to transition the pair of resilient arms from the expanded configuration to the restrained configuration. The finger clip extends between a clip portion fixed to the rolling end portion of the elongate body of the insertion member and a finger portion extending away from the rolling end portion of the elongate body of the insertion member and over at least the first tube end portion of the clamp tube. The finger portion defines a sheet gap between the finger and the clamp tube. The sheet gap is configured to receive and maintain a proximal end of an implantable mesh therein.

Methods of repairing a soft tissue defect, and particularly a hernia such as a ventral hernia are also described.

### Brief Description of the Drawings

Various embodiments of the kits and/or components are described herein with reference to the drawings wherein:
Fig. 1 is top view of a kit described in at least one embodiment herein;
Fig. 2A is a perspective view of a one-piece delivery device described in at least one embodiment herein;
Fig. 2B is a top view of the delivery device of Fig. 2A described in at least one embodiment herein;
Fig. 2C is a side view of the delivery device of Fig. 2A described in at least one embodiment herein;
Fig. 2D is a top view of the delivery device of Fig. 2A in a restrained or closed configuration as described in at least one embodiment herein;
Fig. 3A is a top view of a one-piece delivery device described in at least one embodiment herein;
Fig. 3B is an expanded top view of a portion of the delivery device of Fig. 3A described in at least one embodiment herein;
Fig. 3C is a side view of the delivery device of Fig. 3A described in at least one embodiment herein;
Fig. 3D is an end perspective view of a portion of the delivery device of Fig. 3A as described in at least one embodiment herein;
Fig. 3E is a top view of the delivery device of Fig. 3A in a restrained or closed configuration as described in at least one embodiment herein;
Fig. 4 is a top view of a one-piece delivery device described in at least one embodiment herein;
Fig. 5A is a perspective view of a one-piece delivery device described in at least one embodiment herein;
Fig. 5B is a top view of the delivery device of Fig. 5A described in at least one embodiment herein;
Fig. 5C is a side view of the delivery device of Fig. 5A described in at least one embodiment herein;
Fig. 5D is an end perspective view of a portion of the delivery device of Fig. 5A as described in at least one embodiment herein;
Figs. 6-8D are top views of various delivery devices described in at least one embodiment herein;
Fig. 9A is a perspective view of a two-piece delivery device described in at least one embodiment herein;
Fig. 9B is a top view of the delivery device of Fig. 9A described in at least one embodiment herein;
Figs. 9C-9D are perspective views of opposing end portions of the delivery device of Fig. 9A described in at least one embodiment herein;
Fig. 9E is a side view of the delivery device of Fig. 9A described in at least one embodiment herein;
Fig. 10A is a top view of a first body member of a two-piece delivery device as described in at least one embodiment herein;
Fig. 10B is a front view of a first proximal end portion of the first body member of Fig. 10A as described in at least one embodiment herein;
Fig. 10C is a rear perspective view of a first distal end portion of the first body member of Fig. 10A as described in at least one embodiment herein;
Fig. 10D is a cross-sectional view of a resilient arm of the first body member of Fig. 10A as described in at least one embodiment herein;
Fig. 10E is a side view of a second body member of a two-piece delivery device as described in at least one embodiment herein;
Fig. 10F is a perspective view of a second distal end portion of the second body member of Fig. 10E as described in at least one embodiment herein;
Fig. 10G is a top view of a two-piece delivery device including the first and second body members of Figs. 10A and 10E, respectively, as described in at least one embodiment herein;
Fig. 10H is a side view of the two-piece delivery device of Fig. 10G as described in at least one embodiment herein;
Fig. 10I is perspective view of a proximal end portion of the two-piece delivery device of Fig. 10G as described in at least one embodiment herein;
Fig. 10J is perspective view of a distal end portion of the two-piece delivery device of Fig. 10G as described in at least one embodiment herein;
Fig. 11A is a side view of an insertion member described in at least one embodiment herein;
Figs. 11B-11C are perspective views of opposing end portions of the insertion member of Fig. 11A described in at least one embodiment herein;
Fig. 11D is a side view of a clamp tube of the insertion member of Fig. 11A described in at least one embodiment herein;
Fig. 12A is a side view of an insertion member described in at least one embodiment herein;
Fig. 12B is a perspective view of a portion of the insertion member of Fig. 12A described in at least one embodiment herein;
Figs. 12C-12D are side views of the insertion member of Fig. 12A in combination with a delivery device as described in at least one embodiment herein;
Fig. 13A is a side view of a clamp tube of an insertion member described in at least one embodiment herein;
Fig. 13B is a top view of an insertion member including the clamp tube of Fig. 13A in combination with a delivery device as described in at least one embodiment herein;
Fig. 13C is a side view of an insertion member including the clamp tube of Fig. 13A in combination with a delivery device as described in at least one embodiment herein;
Fig. 14A is a side view of a rolling device described in at least one embodiment herein;
Fig. 14B is a rear view of the rolling device of Fig. 14A as described in at least one embodiment herein;
Fig. 15A is a side view of a rolling device described in at least one embodiment herein;
Fig. 15B is a rear view of the rolling device of Fig. 15A as described in at least one embodiment herein;
Figs. 16A-16Q are schematic images of forming a rolled folded sheet-delivery device assembly as described in at least one embodiment herein;
Figs. 17A-17D are schematic images of inserting a rolled folded sheet-delivery device assembly as described in at least one embodiment herein; and,
Figs. 18A-18O are schematic images of deploying, orienting, and fixating an implantable sheet and removing a delivery device as described in at least one embodiment herein.

### Detailed Description

The present disclosure describes a kit suitable for repairing various soft tissue defects, and particularly for repairing various types of hernias. The kit can include any of the following components, individually or in any combination: An implantable sheet, a central tie, a flexible delivery device, a rolling device, and an insertion member. In some embodiments, the delivery device is a one-piece delivery device. In some embodiments, the delivery device is a two-piece delivery device.

In some embodiments, the kits described herein may include at least an implantable sheet, a central tie, and a flexible one-piece or two-piece delivery device configured to be secured to a portion of the implantable sheet. Such kits may further include a rolling device, an insertion member, or both.

The present disclosure further describes packaging for any of the kits and/or the individual components of the kits described herein. As well as methods of treating or repairing various soft tissue defects or hernias utilizing any of the kits and/or components described herein. Methods of preparing, inserting, orienting, deploying, and/or fixating of an implantable sheet using the various components descried herein are also provided.

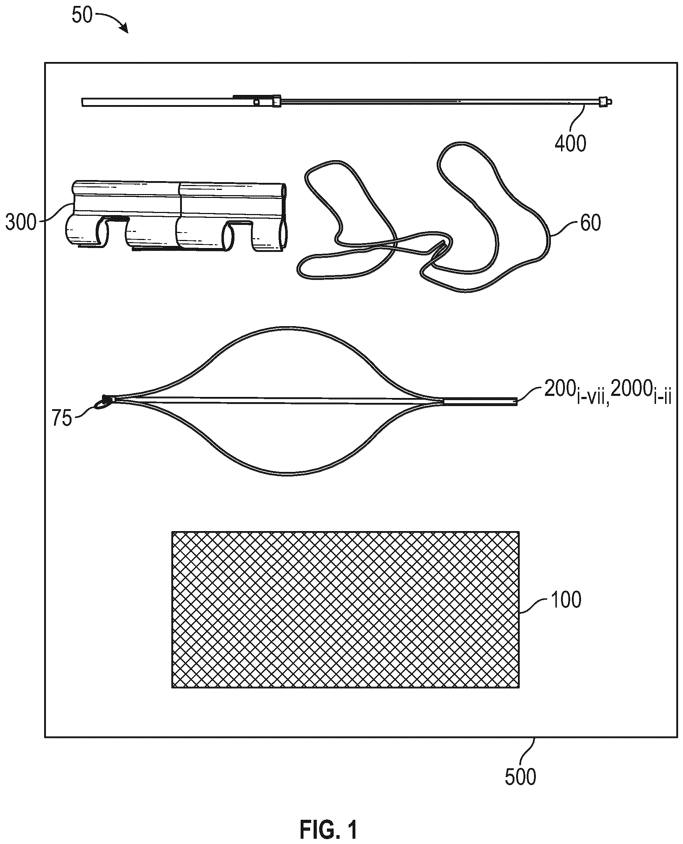

In Fig. 1, a kit 50 as described in at least one embodiment herein is depicted. The kit 50 includes at least an implantable sheet 100, such as a surgical mesh, and a one-piece or multiple-piece delivery device 200*ᵢ₋ᵥᵢᵢ,* 2000*ᵢ₋ᵢᵢ* as described herein. The kit 50 is shown being positioned or stored in a package 500 and further including a rolling device 300, an insertion member 400, and a central tie 60 and a looped material or suture 75 extending from the delivery device 200*ᵢ₋ᵥᵢᵢ,* 2000*ᵢ₋ᵢᵢ.* Each of these components are provided in more detail hereinbelow.

### I.Implantable Sheet

By implantable, the sheets described herein are configured to be positioned at a location within a body for any sufficient amount of time to at least temporarily treat and/or repair a soft tissue defect. In some embodiments, the biocompatible sheet is configured to be located within a portion of the abdominal cavity.

The implantable sheets described herein can be generally planar and may include any biocompatible porous or non-porous material configured to treat and/or repair a soft tissue defect. Some non-limiting examples of suitable sheets include surgical mesh, tissue scaffolds, adhesion barriers, surgical slings, surgical foams, and combinations thereof. The implantable sheet may be woven, non-woven, knitted, braided, cast, extruded, pressed, lyophilized, and the like. The implantable sheet can be bioresorbable, partially bioresorbable or non-bioresorbable.

In some embodiments, the implantable sheets described herein are surgical mesh. In the context of this application the term "mesh", "surgical mesh", or "implantable mesh" refers to an arrangement of biocompatible filaments or yarns, for example a knitted material or woven or nonwoven fibrous material, arranged in a manner to include pores within the mesh face that can encourage tissue ingrowth. The mesh can be bioresorbable, partially bioresorbable or non-bioresorbable. The mesh is generally planar or includes at least a portion which is generally planar. The mesh includes first and second opposite faces and an outer perimeter which defines a center of the mesh on each face. The mesh is also flexible enough to be rolled onto the exterior of the delivery device and upon itself prior to insertion into a patient or a cavity defined within of a patient. The mesh can be produced from one or more layers of fabric and may optionally include an anti-adhesion barrier layer positioned on at least one portion or one side of the fabric thereby forming a composite mesh. Such meshes are well known to the person skilled in the art. The mesh can also be provided in any shape (rectangular, square, circular, oval, etc.) and size. In some embodiments, the mesh may be round or elliptical in shape when unrolled.

The implantable mesh may be a two-dimensional knitted fabric or a three-dimensional knitted fabric. In the context of the present application, the expression "two-dimensional knitted fabric" means a knitted fabric having two opposite faces linked together by stitches but having no spacers imparting a certain thickness to it: such a knitted fabric may be obtained, for example, by knitting threads on a warp or Raschel knitting machine using two guide bars. Examples of two-dimensional knitted fabrics suitable for the present invention are given in the document WO2009/071998.

In the present application, the expression "three-dimensional knitted fabric" means a knitted fabric having two opposite faces linked together by spacers imparting a significant thickness to the knitted fabric, said spacers consisting of connecting threads additional to the threads forming the two faces of the knitted fabric. Such a knitted fabric may be obtained, for example, using a double-bed Raschel knitting machine or warp knitting machine with a plurality of guide bars. Examples of knitting three-dimensional knitted fabrics suitable for the present invention are given in the documents WO99/05990, WO2009/031035, WO2009/071998.

Additionally, meshes within the scope and context of this disclosure may include fibrous biologic materials such as allografts (i.e., AlloDerm® Regenerative Tissue Matrix from Allergan), autografts, and xenografts (i.e., PERMACOL™, from Medtronic).

In some embodiments, the implantable sheets described herein are configured for use in minimally invasive surgical procedures. In some embodiments, the implantable sheets described herein are configured for use with surgical techniques including, but not limited to, TAPPS (transabdominal preperitoneal surgery), TEPS (totally extraperitoneal surgery) or IPOM (intra peritoneal onlay mesh) techniques.

In particularly useful embodiments, the implantable sheet is a surgical mesh or composite surgical mesh suitable for repairing a ventral hernia. In particularly useful embodiments, the implantable sheet is a surgical mesh or composite surgical mesh suitable for repairing a ventral hernia using any appropriate surgical technique, including but not limited to TAPPS, TEPS, or IPOM techniques.

### II. Central Tie

The central tie is designed to connect and/or secure a portion of the delivery device, and particularly a central arm of the delivery device, to the implantable sheet, and particularly a central portion of the implantable sheet, without interfering the movement of the one or more resilient arms.

In some embodiments, the central tie is designed to form at least one loop on one side of the central arm of the delivery device and at least one tie handle extending away from the opposite side of the central arm. The central tie passes through a first and second tie holes defined through the central arm of the delivery device forming a loop between the two tie holes on the one side of the central arm. The central tie also extends from the first and second tie holes and away from a second opposite side of the central arm forming at least one tie handle extending from the delivery device. The at least one tie handle is configured to have a length sufficient to be passed through a thickness of the implantable mesh and also be manipulated from outside the body during implantation. In some embodiments, the central tie (and/or the tie holes) is positioned on or near a center of the length of the central arm. In some embodiments, the central tie is positioned on or near a center of both faces of the implantable sheet.

The central tie can made of any absorbable or nonabsorbable material and has a length greater than its width. For example, the central tie can be in the form of a suture, a fiber, a cable, a chord, a chain, a strip, a ribbon, a tether, a strap, or a long thin tubular mesh.

In some embodiments, the central tie is formed from one suture passing through the delivery device and both sides of the sheet to form the loop and the tie handle. The suture can be bioresorbable, partially bioresorbable or non-bioresorbable. The suture can be barbed or non-barbed. The suture can be armed or unarmed on the ends of the suture handle.

In embodiments wherein the sheet includes an anti-adhesion barrier on at least a central portion thereof, the central tie may also pass through a central portion of the barrier.

The central tie, and particularly the tie handle, is configured to extend from the implantable sheet a length sufficient to be passed from the inside of a cavity of a patient to an outside of the cavity of the patient. In some embodiments, the tie handle is simply formed by the end(s) of the one or more ties extending from the delivery device through the implantable sheet. In some embodiments, the central tie forms two handles extending from the delivery device through the implantable sheet. In some embodiments, the central tie includes one tie handle formed by two or more of the tie ends secured to each other by a knot, crimp, weld, and/or adhesive.

The tie handle is also designed to assist with preparing the sheet for rolling in rolling device, as well as making it simpler to center the sheet on a tissue defect prior to deployment.

In some embodiments, the central tie is added to the delivery device prior to packaging and/or during the manufacturing process of the delivery device. In some embodiments, the central tie may be stored separately in the kit or package and can be added to the delivery device by the surgeon after the package is open. In still other embodiments, the central tie may be a suture packaged separately from the delivery device and added to the delivery device by the surgeon immediately prior to implantation.

In some embodiments, the implantable sheet is an implantable surgical mesh, the central tie is a suture, and the delivery device is one-piece delivery device.

In some embodiments, the implantable sheet is an implantable surgical mesh, the central tie is a suture, and the delivery device is two-piece delivery device.

### III.Delivery Device

The kits described herein include a one-piece or multiple-piece delivery device. It is envisioned that the various elements of the one-piece delivery devices described herein may also be utilized in the two-part delivery devices. The various delivery devices will now be described.

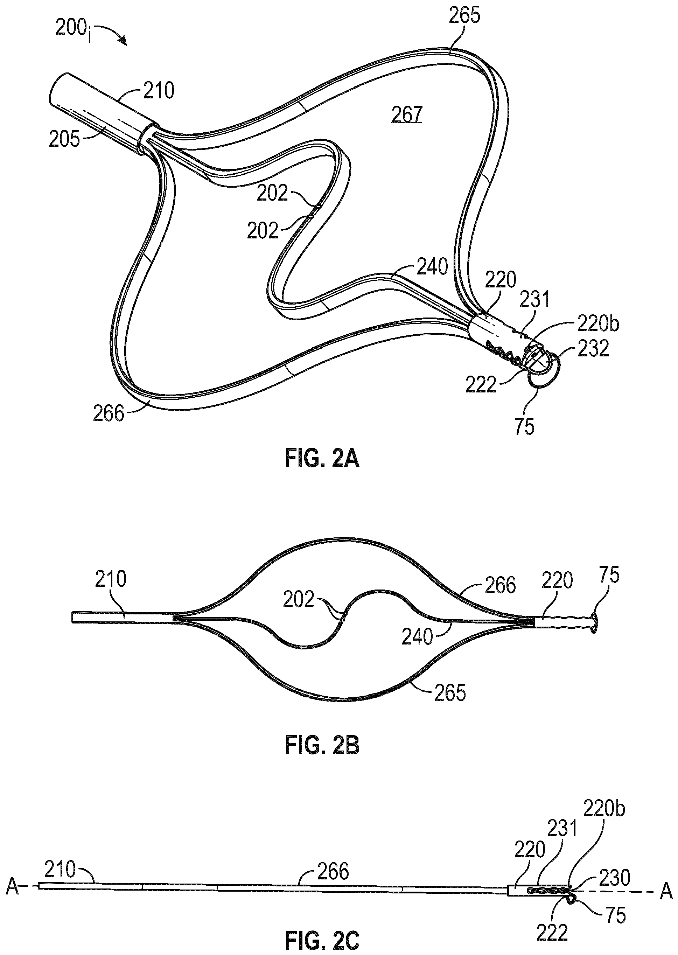

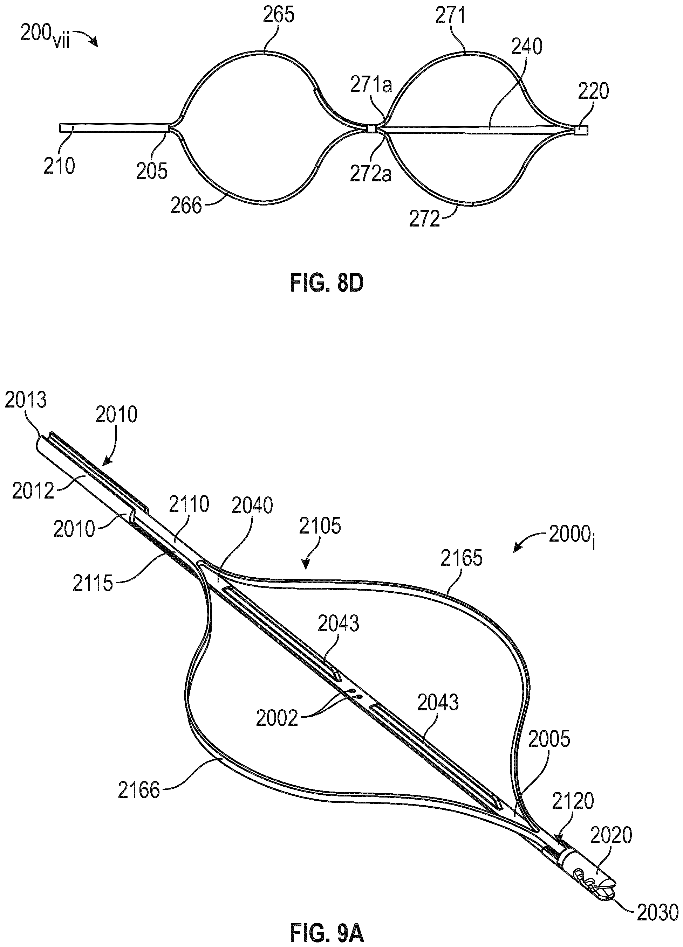

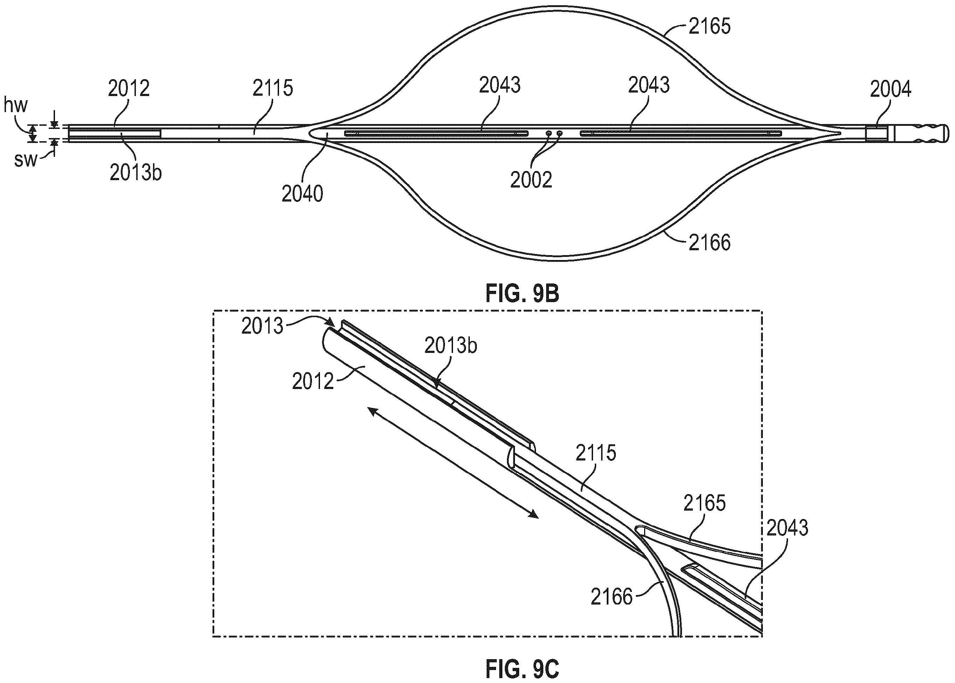

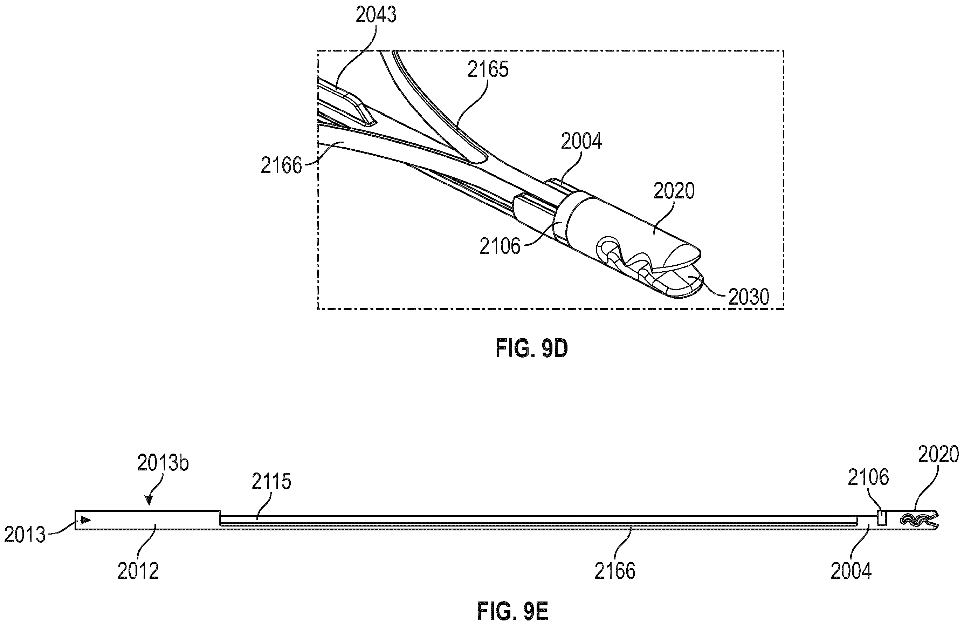

As shown in Figs. 2A-8D, in some embodiments, the delivery device 200*ᵢ₋ᵥᵢᵢ* may include various forms of a one-piece body member 205. As shown in Figs. 9A-10D, in some embodiments, the delivery device may include various forms of a multiple-piece delivery device 2000*ᵢ₋ᵢᵢ* including at least a first and second body member 2005, 2105.

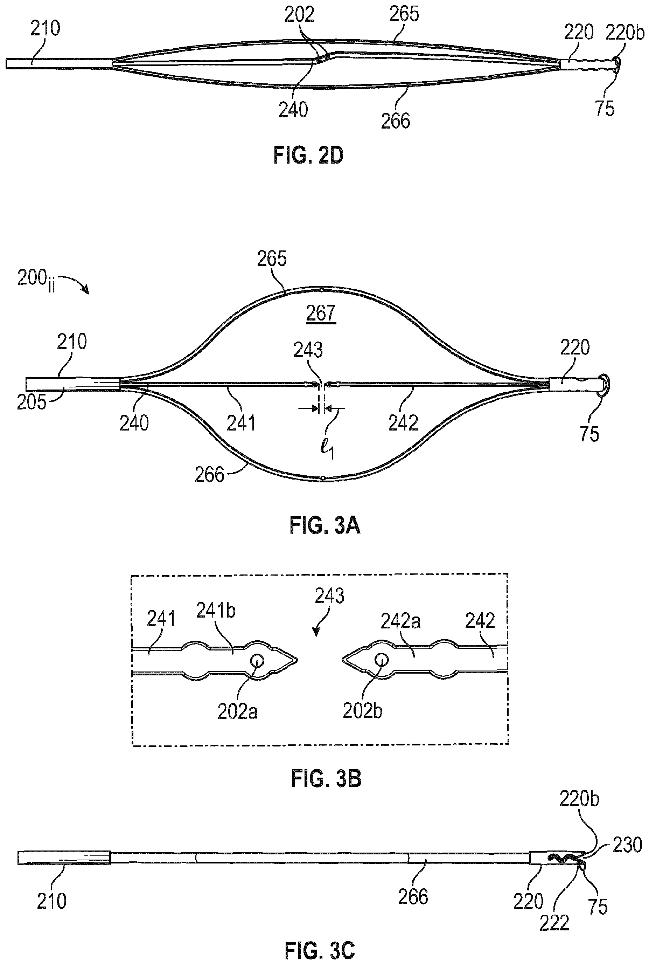

Figs. 2A-2D depict a one-piece delivery device 200*ᵢ* including a one-piece body member 205 extending from a proximal end portion 210 to a distal end portion 220. The body member 205 includes one or more resilient arms 265, 266 and a central arm 240 positioned between the proximal and distal end portions 210, 220 thereof. Both the resilient arms 265, 266 and the central arm 240 may be affixed to both the proximal and distal end portions 210, 220 of the body member 205. The body member 205, and particularly the resilient arms, 265, 266, is configured to transition between an expanded or open configuration (Figs. 2A-2B) with the resilient arms 265, 266 naturally spaced farthest apart from each other (and/or farthest from the central arm 240) and a restrained or closed configuration (Fig. 2D) with the resilient arms generally adjacent each other (and/or adjacent the central arm 240).

In some embodiments, the central arm also may be a resilient arm. As further illustrated in Figs. 2A and 2B, the central arm may be configured to transition from a first non-linear configuration, such as a generally curved or S-shaped configuration, to a generally linear configuration. By generally linear, the resilient arms transition from a predominantly non-linear configuration to a predominantly linear configuration, as depicted in Fig. 2D.

As further shown in Figs. 2A-2B, the central arm 240 also includes a one or more tie holes 202 defined through the central arm 240. The one or more tie holes 202 are configured to allow the central tie 60 described herein to pass therethrough. In some embodiments, a pair of tie holes 202 allow the central tie 60 to enter the body member 205 by passing through the central arm 240 via one of the two tie holes 202 and extending along a length of one side of the central arm 240 before passing through the central arm 240 a second time via the other of the two tie holes 202 forming a loop extending between the two tie holes 202 on the one side of the central arm 240. In some embodiments, the one or more tie holes 202 may be centered on the body member 205.

The proximal end portion 210 of the body member 205 is configured to be received within a first or second socket 415, 425 of the insertion member 400, described in more detail hereinbelow. The distal end portion 220 of the body member 205 is configured to receive and maintain a distal portion of the implantable sheet 100.

As further provided in Figs. 2A-2C, the delivery devices 200*ᵢ* described herein also include a distal end portion 220 including a slot 230 extending proximally from the distal end 220b of the body member 205. The slot 230 divides at least a part of the distal end portion 220 into an upper jaw member 231 and a lower jaw member 232. The slot 230 is configured to receive and secure at least a distal portion of an implantable sheet between the upper and lower jaw members 231, 232. In some embodiments, the slot 230 is configured to receive and secure a distal end portion of a sheet 100 which can be folded over one of the upper or lower jaw members 231, 232, as well as the distal end 220b of the delivery device 200*ᵢ*.

In some embodiments, the slot 230 extends generally along a central longitudinal axis A₁ of the distal end portion 220 of the body member 205, thereby dividing the distal end portion 220 into symmetrical upper and lower jaw members 231, 232. However, it is envisioned that in some embodiments, the slot may be offset from the central longitudinal axis dividing the distal end portion into asymmetrical upper and lower jaw members.

As shown, the slot 230 defines a wavy or sinusoidal pathway through the distal end portion 220 of the delivery device 200*ᵢ*. In some embodiments, the slot may be generally linear as compared to wavy or sinusoidal. The wavy or sinusoidal pathway provides a greater surface area to the slot 230 thereby increasing the surface area in contact with the implantable sheet when positioned in the slot 230. This increased surface area improves the hold strength of the slot 230 on the sheet, as compared to a slot having a generally linear pathway. The number and/or size of the waves can vary to optimize the hold strength of the delivery device.

In addition to being configured to receive and retain a portion of the implantable sheet within the slot 230 defined in the distal end portion 220 of the delivery device 200*ᵢ*, the upper and lower jaw members 231, 232 also may be flexible. Therefore, the jaw members 231, 232 possess a naturally flexibility that can be used to either expand the size of the slot 230 by forcing the jaw members 231, 232 away from each other to remove the implantable sheet from the slot or decrease the size of the slot 230 by forcing the jaw members 231, 232 towards each other thereby pinching the jaw members 231, 232 onto the sheet positioned therebetween.

The distal end portion 220 of the delivery devices 200*ᵢ* described herein also include a suture aperture 222 configured to receive a looped material, such as suture or looped suture 75. In some embodiments, the suture aperture 222 is positioned on or near the most distal portion of the delivery device 200*ᵢ* to make it easily accessible when inside the patient because the suture aperture 222 and/or the looped material positioned therein is configured to lead the way when the delivery device 200*ᵢ* is withdrawn from a patient. The suture or looped suture 75 positioned within and extending from the suture aperture 222 also provides a larger target to grasp when trying to retrieve the delivery device 200*ᵢ*, as compared to the rounded distal end portion 220 of the delivery device 200*ᵢ* without a suture or suture loop.

Turning to Figs. 3A-3C, in some embodiments, the one-piece delivery device 200*ᵢᵢ* includes a one-piece body member 205 extending from a proximal end portion 210 to a distal end portion 220. The body member 205 includes a pair of resilient arms 265, 266 and a central arm 240 positioned between the proximal and distal end portions 210, 220 thereof. Both the resilient arms 265, 266 and the central arm 240 may be affixed to both the proximal and distal end portions 210, 220 of the body member 205. The body member 205, and particularly the resilient arms, 265, 266, is configured to transition between an expanded or open configuration (Fig. 3A) with the resilient arms 265, 266 naturally spaced farthest apart from each other (and/or farthest from the central arm 240) and a restrained or closed configuration with the resilient arms generally adjacent each other (and/or adjacent the central arm 240).

As shown in Figs. 3A-3B, in some embodiments, the central arm 240 may include a central gap 243 positioned between a first arm part 241 and a second arm part 242 of the central arm 240. The first arm part 241 and the second arm part 242 may be spaced longitudinally by the central gap 243. The first arm part 241 is affixed to and extends distally from the proximal end portion 210 of the body member 205. The second arm part 242 is affixed to and extends proximally from the distal end portion 220 of the body member 205.

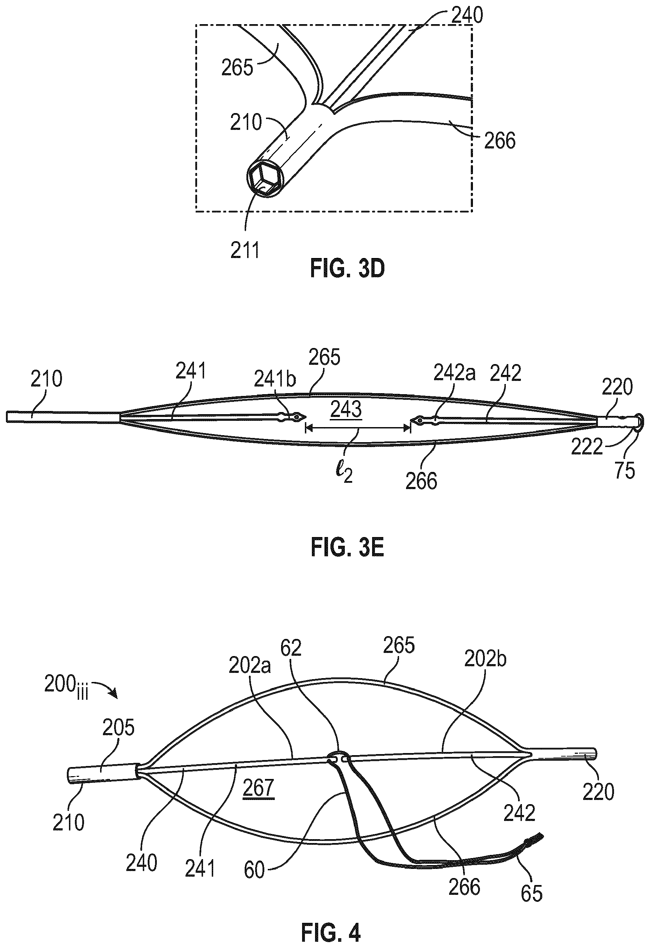

The first arm part 241 and the second arm part 242 are closest to each other in the expanded or open configuration shown in Fig. 3A. In the expanded or open configuration, the central gap 243 defines a first length l₁ which is narrower than in the restrained or closed configuration. The first arm part 241 and the second arm part 242 are farthest from each other in the restrained or closed configuration shown in Fig. 3E. In the restrained or closed configuration, the central gap 243 defines a second length l₂ which is greater than the first length l₁ in the expanded or open configuration. As the delivery device transitions from the expanded configuration to the restrained configuration, the resilient arms 265, 266 are forced towards each other causing the proximal and distal end portion 210, 220 to move away from each other longitudinally causing the central gap 243 to widen as the first and second arm parts 241, 242 are forced away from each other by the proximal and distal end portions 210, 220, respectively.

As further shown in Figs. 3A-3B, the central arm 240 may include a first and second tie hole 202a, 202b defined therethrough. In some embodiments, the first tie hole 202a may be defined through a distal end portion 241b of the first part 241 of the central arm 240 and the second tie hole 202b may be defined through a proximal end portion 242a of the second part 241 of the central arm 240. The central gap 243 being positioned between the first and second tie holes 202a, 202b.

In some embodiments, as shown in Fig. 3D, the proximal end portion 210 may be round or circular in shape and include an indentation 211 into a thickness of the proximal end portion 210 of the body member 205. The indentation 211 provides an additional position for the first and/or second sockets of the insertion member to interact and lock into place with when secured to the proximal end portion 210 of the delivery device 200*ᵢᵢ*.

In some embodiments, the shape of the indentation and the shape of the proximal end portion of the body member may be the same. In some embodiments, the shape of the indentation and the shape of the proximal end portion may be different (Fig. 3D).

As depicted, the indentation 211 may be hexagonal. However, other various shapes of the indentation 211 (or the matching protrusion on the insertion member) are also envisioned including, but not limited to circular-shaped, elliptical-shaped, triangular-shaped, square-shaped, rectangular-shaped, pentagonal-shaped, octagonal-shaped, star-shaped, cross-shaped, and the like.

As further provided in more detail in Figs. 3A and 3D, the delivery devices described herein also include a distal end portion 220 which includes a suture aperture 222 configured to receive a looped material, such as suture or looped suture 75.

In some embodiments, the first arm part 241 and the second arm part 242 of the central arm 240 may be a stiff rod or band. In some embodiments, the central arm may be made of a resilient material. In some embodiments, the central may be made of a stiff, non-resilient material.

As depicted in Figs. 2A and 3A, in some embodiments, the pair of resilient arms 265, 266 may naturally define a generally eye shaped opening 267 therebetween in the expanded or open configuration. However, it is envisioned that the resilient arms can be configured to form a variety of differently shaped openings including, but not limited to, square, triangular, octagonal, hexagonal, elliptical, and the like. For example, as depicted in Fig. 4, the resilient arms 265, 266 may naturally define a generally elliptical opening 267 therebetween in the expanded or open configuration.

As further depicted in Fig. 4, the central tie 60 extends through the first and second tie holes 202a, 202b forming a loop 62 through the central arm 240 and a tie handle 65 extending away from the delivery device 200*ᵢᵢᵢ.*

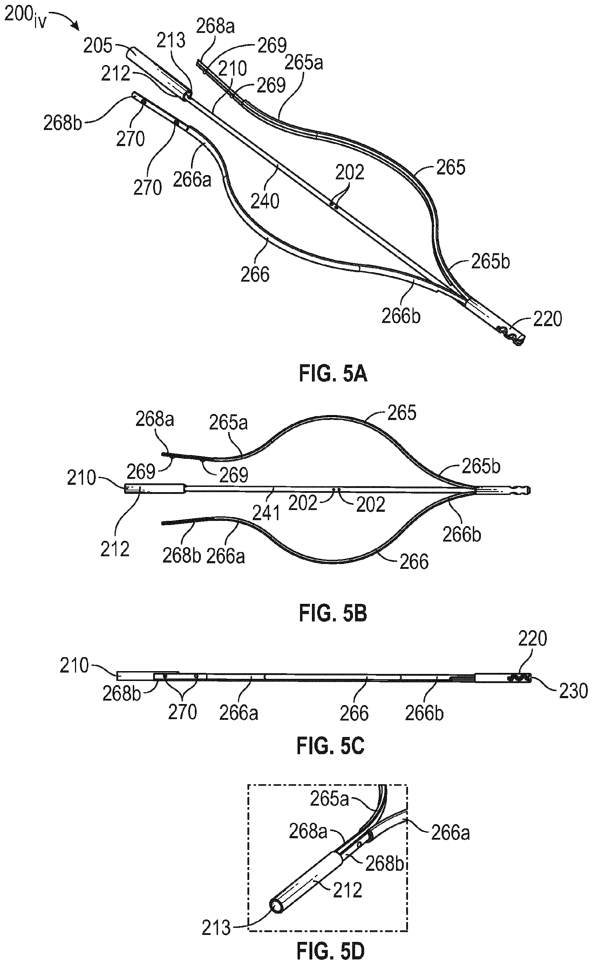

As shown in Figs. 5A-5D, in some embodiments, one-piece delivery device 200*ᵢᵥ* may include central arm 240 affixed to both the proximal and distal end portions 210, 220 of the body member 205 while the distal end portions 265b, 266b of the resilient arms 265, 266 are affixed to only the distal end portion 220 of the body member 205. The proximal end portions 265a, 266a of the resilient arms 265, 266 remain free of or not fixed to the proximal end portion 210 of the body member 205. However, the proximal end portions 265a, 266a of the resilient arms 265, 266 may further include an alignment post 268a, 268b extending therefrom and configured to be received within a tube 212 defined on the proximal end portion 210 of the body member 205.

Each of the alignment posts 268a, 268b may be narrower than the resilient arms 265, 266 and configured to slide within a tube lumen 213 of the tube 212 when the resilient arms 265, 266 transition between an expanded or open configuration and a restrained or closed configuration as described herein.

In addition, in some embodiments, one of the alignment posts 268a may further include one or more alignment tabs 269 extending therefrom and configured to be received and maintained within the one or more alignment tab recesses 270 defined through the other of the alignment posts 268b. As depicted in Fig. 5D, the alignment posts 268a, 268b of the resilient arms 265, 266 can be aligned with and/or secured to each other by inserting the alignment tabs 269 into the alignment tab recesses 270 a prior to insertion into the tube 212. The tube 212 being fixed to the proximal end portion 210 of the body member 205.

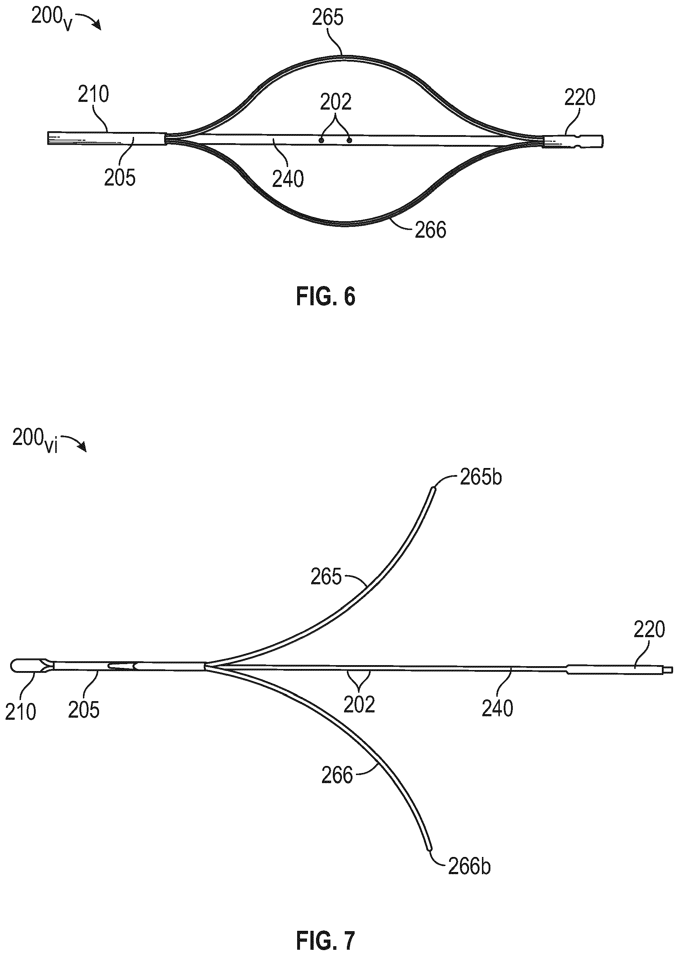

Figs. 6-8D depict various other delivery device 200*ᵥ₋ᵥᵢᵢ* configurations. Although referenced as one-piece delivery devices, each of the delivery devices of Figs. 6-8D may alternatively be two-piece delivery devices.

As illustrated in Fig. 6, in some embodiments, the delivery device 200*ᵥ* includes a central arm 240 in a different plane than the resilient arms 265, 266. For example, the resilient arms 265, 266 may be positioned on top of the wide flat band of a central arm 240, as opposed to being positioned in the same plane as the central arm, as shown in for example Figs. 2A-2D.

In Fig. 7, in some embodiments, the resilient arms 265, 266 and the central arm 240 are both affixed to the proximal end portion 210 of the delivery device 200*ᵥᵢ* while the resilient arms 265, 266 are free of and/or not affixed to the distal end portion 220 of the body member 205. Since one end, e.g., the distal end portion 220 in Fig. 7, is configured to remain free of and/or unattached to the body member 205, the resilient arms 265, 266, in some embodiments, may not define an opening having a closed shape such as an eyeball, ellipse, etc.

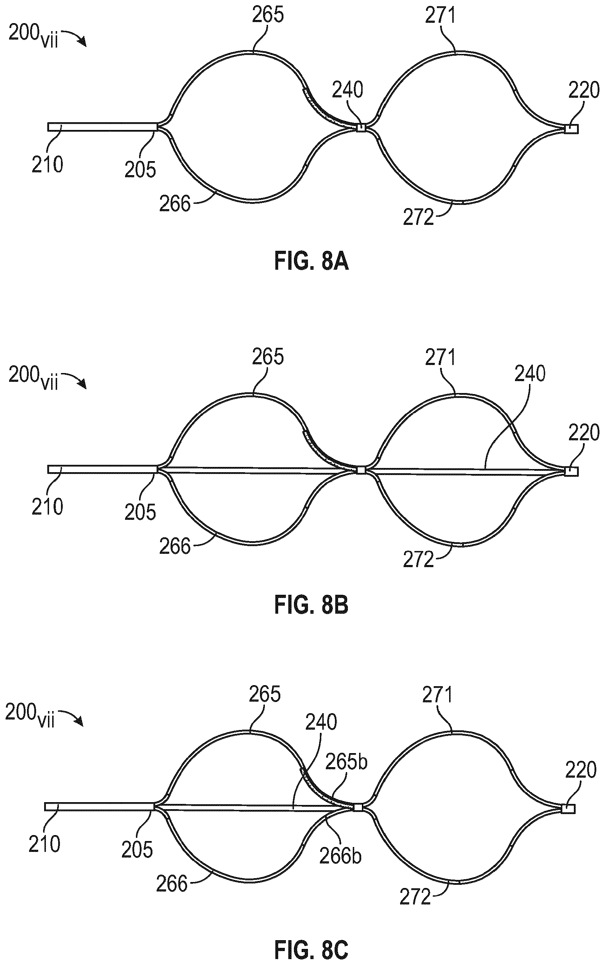

In Figs. 8A-8D, in some embodiments, the delivery devices described herein may include a plurality of resilient arms 265, 266, 271, 272. For example, in Figs. 8A-8D, a first pair of resilient arms 265, 266 are affixed to the proximal end portion 210 of the delivery device 200*ᵥᵢᵢ* and a second pair of resilient arms 271, 272 are affixed to the distal end portion 220 of the delivery device 200*ᵥᵢᵢ*. In some embodiments, as shown in Fig. 8A, the delivery device 200*ᵥᵢᵢ* may be free of a central arm. In some embodiments, as shown in Fig. 8B, the delivery device 200*ᵥᵢᵢ* may include a central arm 240 positioned at least between the first and second pairs of resilient arms 265, 266, 271, 272. In some embodiments, the central arm 240 may only extend from the proximal end portion 210 to the distal end portions 265b, 266b of the first pair of resilient arms 265, 266 (Fig. 8C). In some embodiments, the central arm 240 may only extend from the proximal end portions 271a, 272a of the second pair of resilient arms 271, 272 to the distal end portion 220 of the body member 205 (Fig. 8D). In each of these embodiments, the central arm 240 may be free of one or more of, if not both, the first and second end portion 210, 220 of the body member 205. In each of these embodiments, the central arm 240 may be affixed to one or more of, if not both, the first and second end portion 210, 220 of the body member 205.

In some embodiments, the delivery device may be a two-piece delivery device. The two-piece delivery includes first and second body members configured to attach and/or lock to each other to form a delivery device as described herein. The two-piece delivery devices described herein, like the one-piece delivery devices described herein, are configured to deliver an implantable sheet, such as a surgical mesh into a patient. Some examples of two-piece delivery devices will be described further below.

As shown in Figs. 9A-9E, in some embodiments, the one or more resilient arms 2165, 2166 and the central arm 2040 are located on two different body members 2005, 2105 of the two-part delivery device 2000*ᵢ*. Particularly, a first body member 2005 of the delivery device 2000*ᵢ* includes a central arm 2040 extending between a first proximal end portion 2010 and a first distal end portion 2020, and a second body member 2105 of the delivery device 2000*ᵢ* includes one or more resilient arms 2165, 2166 extending between a second proximal end portion 2110 and a second distal end portion 2120. The first distal end portion 2020 and the second distal end portion 2120 being configured to attach to and/or lock to each other to form the delivery device 2000*ᵢ*.

As further depicted in Figs. 9A-9C, the first proximal end portion 2010 of the first body member 2005 includes a first tube 2012 defining a first tube lumen 2013 therein and the second proximal end portion 2110 of the second body member 2100 includes a shaped handle 2115 configured to be received and/or maintained within the first tube lumen 2013 of the first tube 2012. As indicated by the arrow in Fig. 9C, the shaped handle 2115 is also configured to be movable and/or slidable longitudinally within the first tube lumen 2013. Such movement and/or sliding is associated with the transitioning of the resilient arms between an expanded configuration and a restrained configuration.

In some embodiments, the first tube 2012 of the first body member 2005 further includes a tube slot 2013b in communication with the first tube lumen 2013. The tube slot 2013b, like the first tube lumen 2013, may extend the entire length of the first tube 2012. The tube slot 2013b defines a slot width sw which smaller than a handle width hw of the handle 2115 to ensure the handle 2115 is prevented from being dislodged from the first tube lumen 2013 through the tube slot 2013b.

As further depicted in Figs. 9A-9B and 9D, in some embodiments, the first distal end portion 2020 of the first body member 2005 includes a body locking recess 2004 defined therein and the second distal end portion 2120 of the second body member 2105 includes a locking member 2106 configured to be received within the body locking recess 2004 to attach and/or lock the first and second distal end portions 2020, 2120 to each other to form the delivery device 2000*ᵢ*. By locking the first and second distal end portions 2020, 2120 together, the proximal handle 2115 remains free to slide within the tube 2012 enabling the delivery device 2000*ᵢ* to transition between an expanded configuration and a restrained configuration. The body locking recess 2004 may be located proximal to the slot 2030 configured to receive an implantable sheet therein.

In some embodiments, the shape of the body locking recess 2004 may be generally T-shaped. In some embodiments, the locking member 2106 may be generally T-shaped to matingly engage a T-shaped body locking recess 2004.

As still further depicted in Figs. 9A-9B and 9D, the central arm 2040 may include one or more raised support members 2043 extending therefrom. The raised support members 2043 and the central arm 2040 forming a generally T-shaped cross-section. In some embodiments, the central arm 2040 may include a pair of raised support members 2043 separated by a pair of tie holes 2002 positioned between.

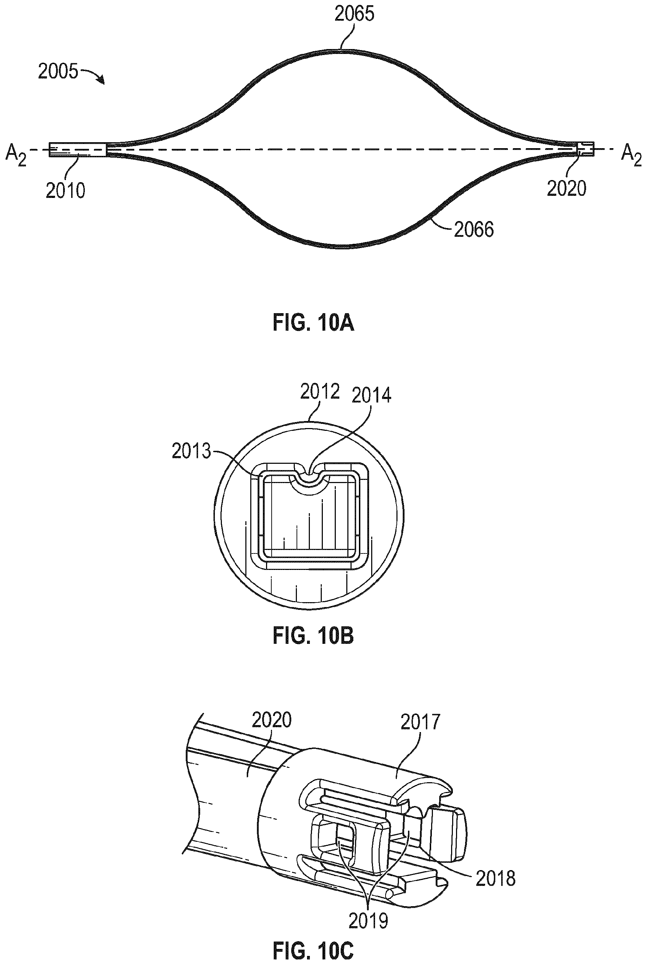

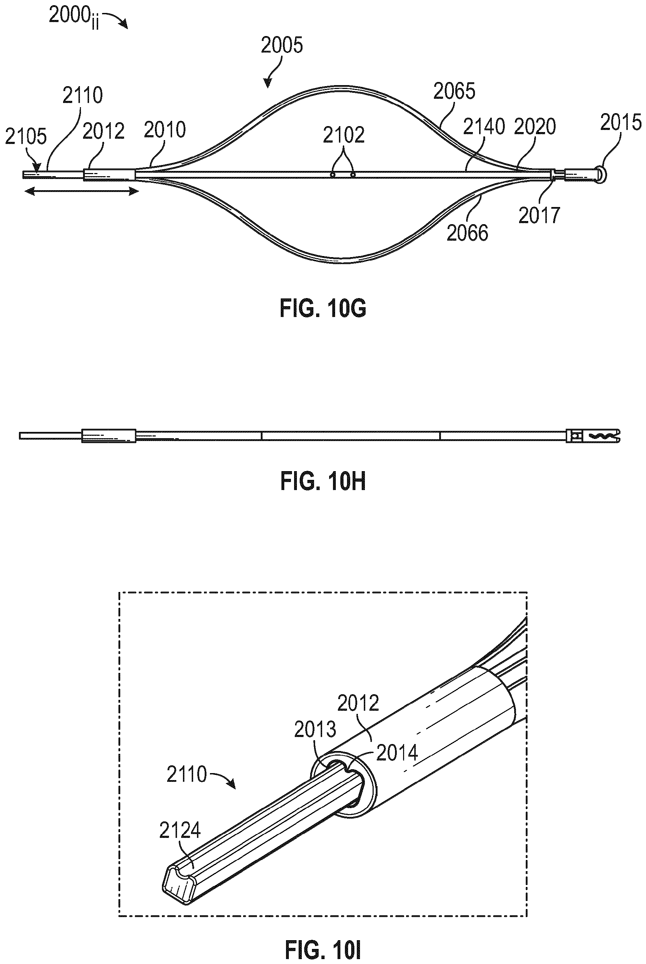

As shown in Figs. 10A and 10B, in some embodiments, the one or more resilient arms 2065, 2066 and the central arm 2140 may be located on two different body members 2005, 2105 of the two-part delivery device 2000*ᵢᵢ*. Particularly, a first body member 2005 of the delivery device 2000*ᵢᵢ* includes one or more resilient arms 2065, 2066 extending between a first proximal end portion 2010 and a first distal end portion 2020, and a second body member 2105 of the delivery device 2000*ᵢᵢ* includes a linear central arm or rod 2140 extending between a second proximal end portion 2110 and a second distal end portion 2120. The first distal end portion 2020 and the second distal end portion 2120 being configured to attach to and/or lock to each other to form the delivery device 2000*ᵢᵢ*.

As further depicted in Figs. 10A-10C, the first body member 2005 includes a first and second tube 2012, 2017 fixed on opposite end portions of the first body member 2005 with the resilient arms 2065, 2066 affixed therebetween. Particularly, the first proximal end portion 2010 of the first body member 2005 includes a first tube 2012 defining a first tube lumen 2013 therethrough and the first distal end portion 2020 includes a second tube 2017 defining a second tube lumen 2018 therethrough. Although the first and second tubes 2012, 2017 are spaced apart from each other, the first and second tubes 2012, 2017, as well as the first and second tube lumens 2013, 2018, are aligned with the same longitudinal axis A₂.

In some embodiments, the first and second tube lumens 2013, 2018 define the same cross-sectional shape. For example, as shown in Figs. 10B-10C, the first and second tube lumens 2013, 2018 may define the same generally square cross-section. It is envisioned that any cross-sectional shape may be utilized including but not limited to circular, elliptical, rectangular, triangular, pentagonal, hexagonal, heptagonal, octagonal, star-shaped, cross-shaped, and combination thereof.

In addition, the first and/or second tube lumen 2013, 2018 may further include one or more guide members 2014 extending a length of the first and/or second tube lumens 2013, 2018 and protruding into at least one side of the cross-section of the tube lumens 2013, 2018. For example, as further depicted on Figs. 10B-10C, a rounded guide member 2014 extends a length of the first and second tube lumens 2013, 2018 and protrudes into at least one side of the generally square cross-section. It is envisioned that the guide member may be of any shape or design.

The second tube 2017 of the first body member 2005 further includes one or more body locking recesses 2019 defined therein. Each of the locking recesses 2019 is configured to receive a locking member 2119 located on the second body member 2105 to lock the first body member 2005 to the second body member 2105.

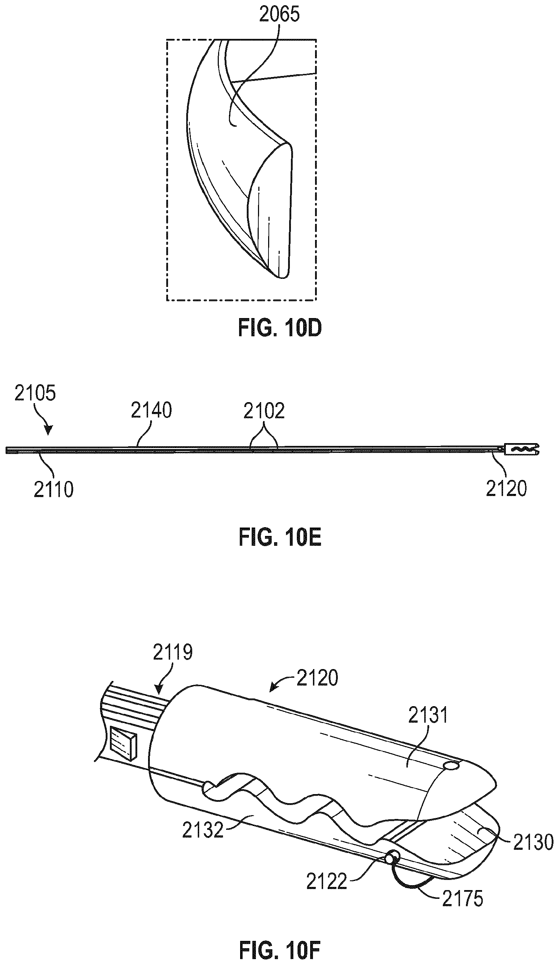

As shown in Fig. 10D, in some embodiments, each of the one or more resilient arms may define a half-moon cross-section.

In Fig. 10E, the second body member 2105 may be generally an elongate body extending between the second proximal end portion 2110 to the second distal end portion 2120. A majority of the second body member 2105, including at least a portion of the second proximal end portion 2110, may be a linear arm or rod configured to be received within the first and second tubes 2012, 2017 (and/or the first and second tube lumens 2013, 2018) to form the delivery device 2000*ᵢᵢ*. The linear arm or rod 2140 also includes a pair of tie holes 2102 defined therethrough.

As illustrated in Fig. 10F, the second distal end portion 2120 may include one or more locking members 2119 extending therefrom, a suture aperture 2122 configured to receive looped suture 75 therein, and a slot 2130 located distal the locking member(s) 2119. The locking member(s) 2119 is configured to be received within the one or more locking recesses 2019 of the first body member 2005. The slot 2130 separates the second distal end portion 2120 into an upper and lower jaw members 2131, 2132. The slot 2130 is configured to receive and secure a distal end portion of an implantable sheet between the upper and lower jaw members 2131, 2132.

As depicted in Fig. 10G, when the first body member 2005 and the second body member 2105 are locked to each other, the second proximal end portion 2110 of the second body member 2105 extends proximally beyond to the first tube 2012 of the first body member 2005. In this configuration, the first tube 2012 is configured to slide and/or move longitudinally (as indicated by the arrow) along the second proximal end portion 2110 as the resilient arms 2065, 2066 transition between the expanded configuration (Fig. 10G) and the restrained configuration.

In some embodiments, a majority of the second body member 2105, particularly at least the second proximal end portion 2110, defines the same cross-sectional shape as the tube lumens 2013, 2018 of the first body member 2005. For example, as shown in Figs. 10I, the second proximal end portion 2110 may define the same generally square cross-sectional shape as the first and/or second tube lumen 2013, 2018. It is envisioned that the second proximal end portion 2110 may be any cross-sectional shape including but not limited to circular, elliptical, rectangular, triangular, pentagonal, hexagonal, heptagonal, octagonal, star-shaped, cross-shaped, and combination thereof.

In addition, a majority of the second body member 2105, particularly at least the second proximal end portion 2110, may further include one or more grooves 2124 extending a length of at least the second proximal end portion 2110 and protruding into at least one side of the cross-section. The groove 2124 configured to accommodate the guide member 2014 of the first body member 2005. For example, as further depicted on Figs. 10I, a rounded groove 2124 extends a length of one side of the generally square cross-sectional shape of the second proximal end portion 2110 and protrudes into at least one side of the generally square cross-section. It is envisioned that the groove 2124 may be of any shape or design.

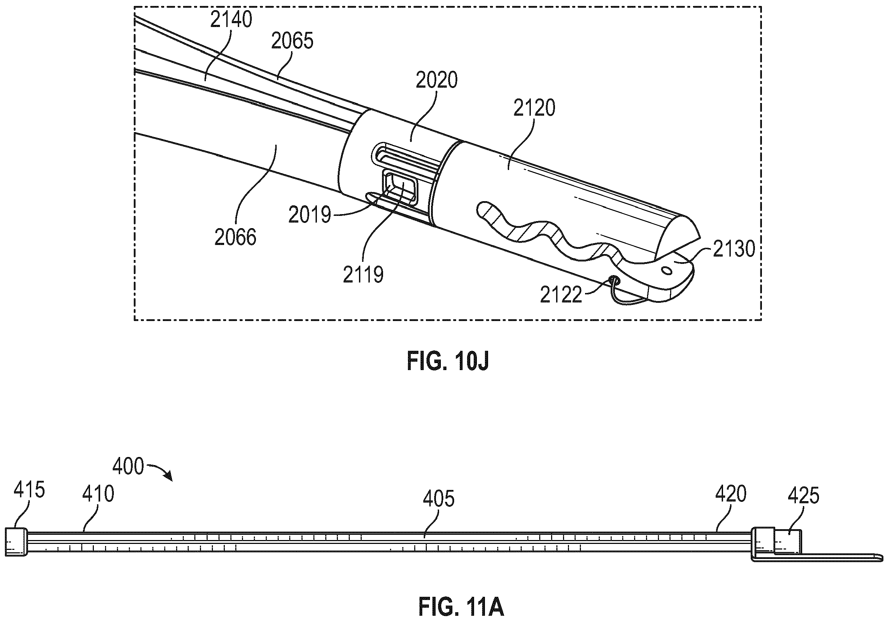

Fig. 10J shows the second distal end portion 2120 of the second body member 2105 locked to the first distal end portion 2020 of the first body member 2005. Specifically, the one or more locking members 2119 are received or snapped into the one or more locking recesses 2019 to affix the two body members together to form the delivery device 2000.

The delivery devices described herein can be made of any biocompatible material. Some non-limiting examples of suitable materials include polyamides, polyaryl ether ketone (PAEK), acrylonitrile butadiene styrene (ABS), polyether ether ketone (PEEK), polyoxymethylene (POM), nitinol (NiTi), polyetherimide (PEI), polycarbonates (PC), and combinations thereof. In addition to being biocompatible the materials used to form the delivery device can be compatible with injection molding manufacturing processes and compatible with standard sterilization methods, such as Ethylene Oxide and gamma radiation.

### IV. Insertion Member

The insertion members described herein are configured to connect or attach to the one-piece or two-piece delivery devices described herein. The insertion member may provide multiple functions. For example, the insertion member may: transition the delivery devices (and/or resilient arms) between an expanded or open configuration and a restrained or closed configuration; roll the implantable sheet on the delivery device in the restrained or closed configuration; and insert the combined implantable sheet and delivery device into a patient.

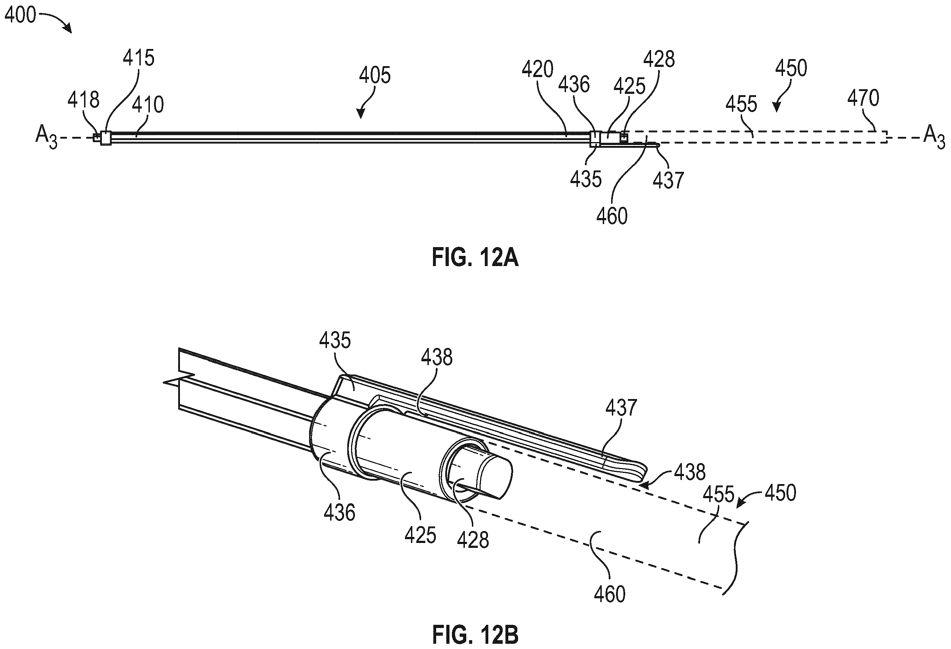

As shown in Figs. 11A-12D, the insertion members 400 described herein include an elongate body 405 and a clamp tube 450 extending therefrom. In Figs. 11A-11D, the elongate body 405 and the clamp tube 450 are shown in separate pieces for clarity purposes. In Figs. 12A-12D, the elongate body 405 and the clamp tube 450 are shown together to form the insertion member 400 with the clamp tube 450 extending from the elongate body 405 along the same longitudinal axis A₃.

The elongate body 405 of the insertion member 400 extends between an insertion end portion 410 and an opposite rolling end portion 420, each of which are individually configured to attach to and/or lock onto the proximal end portions of any of the one-piece or two-piece delivery devices (and/or any of the body members) described herein.

The inserting end portion 410 of the insertion member 400 includes a first socket 415. The first socket 415 is configured to attach to and/or lock onto the proximal end portions of any of the one-piece or two-piece delivery devices (and/or any of the body members) described herein when inserting the delivery device into a patient.

The rolling end portion 420 of the insertion member 400 includes a second socket 425. The second socket 425 is configured to attach to and/or lock onto the proximal end portions of any of the one-piece or two-piece delivery devices (and/or any of the body members) described herein when transitioning the delivery devices (and/or resilient arms) between an expanded or open configuration and a restrained or closed configuration and/or when rolling the implantable sheet on the delivery device in the restrained or closed configuration.

In some embodiments, the first and/or second sockets include a socket recess configured to receive a proximal end portion of the delivery device therein (Figs. 11A-11C). In some embodiments, the first and/or second sockets include a socket protrusion configured to be received within a proximal end portion of the delivery device (Figs. 12A-12D).

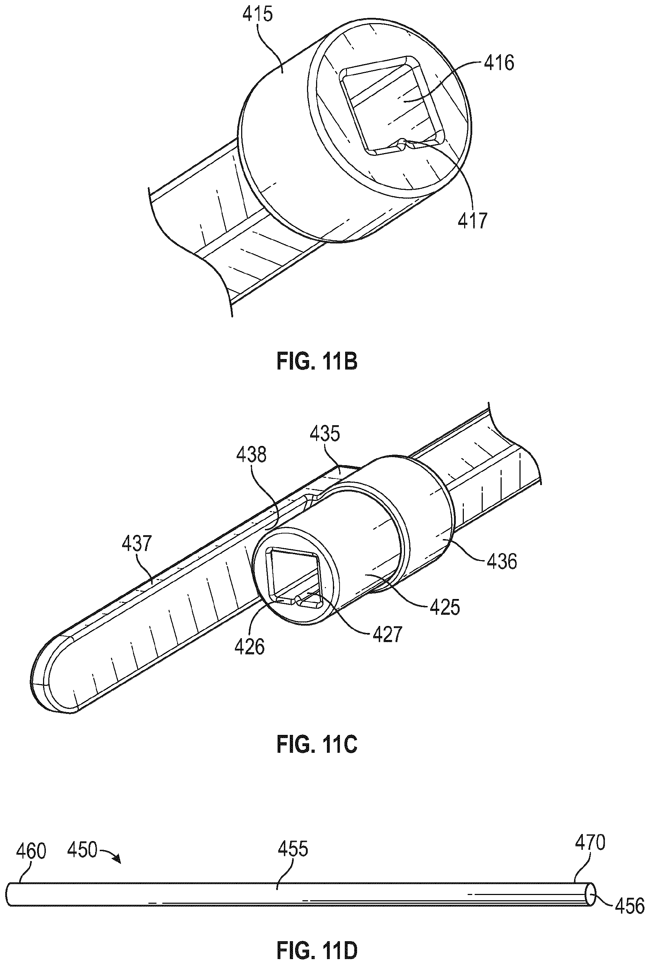

As depicted in Figs. 11B and 11C, in some embodiments, the first and second sockets 415, 425 may include a first and second socket recess 416, 426, respectively. Each socket recess 416, 426 is configured to receive therein a proximal end portion of the delivery device. For example, each of the first and second socket recesses 416, 426 may be generally square-shaped recesses including a recess guide member 417, 427 in order to matingly engage a proximal end portion of one of the body members described herein, such as the proximal end portion 2110 of the second body member 2100 shown in Fig. 10I. Like the proximal end portions described herein, the socket recesses may define any shaped cross-section including circular-shaped, elliptical-shaped, triangular-shaped, square-shaped, rectangular-shaped, pentagonal-shaped, hexagonal-shaped, octagonal-shaped, star-shaped, cross-shaped, and the like. However, in some embodiments, the shaped cross-sections of the proximal end portions and the socket recesses are configured to be complimentary to form a proper male/female connection.

As depicted in Figs. 12A-12D, in some embodiments, the first and second sockets 415, 425 may include a first and second socket protrusion 418, 428, respectively. Each socket protrusion 418, 428 is configured to be received within a proximal end portion of the delivery device. For example, each of the first and second socket protrusions 418, 428 may be a generally rectangular-shaped recesses in order to matingly engage a proximal end portion of one of the body members described herein, such as the rectangular-shaped proximal end portion 210 of the body member 205 shown in Fig. 5D. Like the proximal end portions described herein, the socket protrusions described herein may define any shaped cross-section including circular-shaped, elliptical-shaped, triangular-shaped, square-shaped, rectangular-shaped, pentagonal-shaped, hexagonal-shaped, octagonal-shaped, star-shaped, cross-shaped, and the like. However, in some embodiments, the shaped cross-sections of the proximal end portions and the socket protrusions are configured to be complimentary to form a proper female/male connection.

The rolling end portion 420 of the insertion member 400 also includes a finger clip 435 attached thereto and extending therefrom. The finger clip 435 includes a clip portion 436 and a finger portion 437. The clip portion 436 secures the finger clip 435 to the insertion member 400. The finger portion 437 extends beyond the rolling end portion 420 of the insertion member 400. The finger portion 437 is also spaced from the insertion member 400 and/or the clamp tube 450 creating a finger gap 438 therebetween. The finger gap 438 configured to receive and maintain a proximal end of the implantable sheet during rolling.

As shown in Figs. 11D, 12A, and 12B, the clamp tube 450 has a tubular body 455 extending between a first clamp tube end portion 460 and an opposite second clamp tube end portion 470. The tubular body 455 defining a rolling lumen 456 therethrough. The first clamp tube end portion 460 is affixed to the rolling end portion 420 of the elongate body 405 distal the finger clip 435. In some embodiments, the first clamp tube end portion 460 may be affixed to an outer perimeter of the second socket 425.

The second clamp tube end portion 470 is free of the elongate body 405. The clamp tube 450, and particularly the second clamp tube end portion 470, is configured to cover the proximal end portion of any of the delivery devices described herein, as well as at least some portion of the resilient arms sufficient to restrain the resilient arms in a closed or restrained configuration.

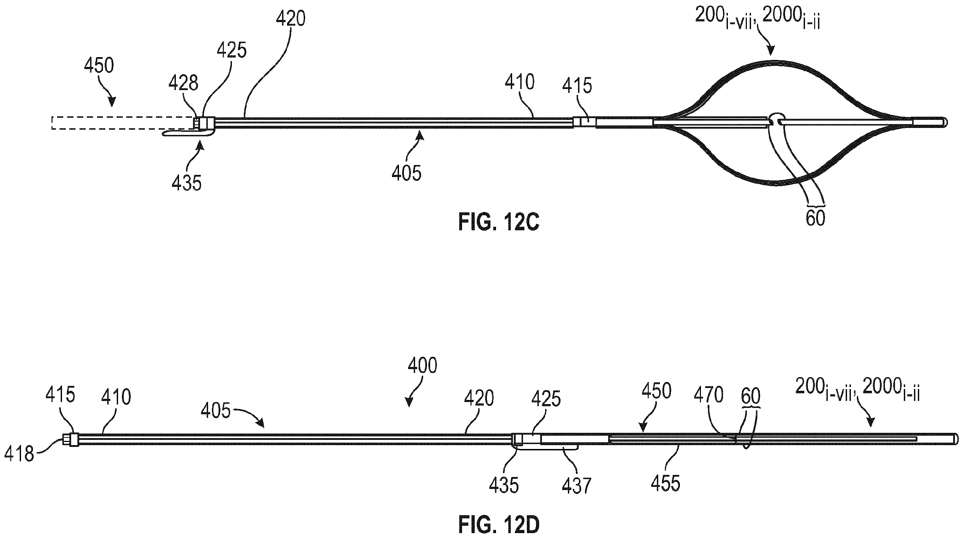

As shown in Fig. 12C, in some embodiments, the insertion end portion 410 via the first socket 415 may be attached to the proximal end portion 210, 2010, or 2110 of any of the delivery devices (and/or body members) described herein. Since the clamp tube 450 extends from the opposite rolling end portion 420 of the elongate body 405, the delivery device 200*ᵢ₋ᵥᵢᵢ,* 2000*ᵢ₋ᵢᵢ* is depicted in an expanded or open configuration.

As shown in Fig. 12D, in some embodiments, the rolling insertion end portion 420 via the second socket 425 may be attached to the proximal end portion 210, 2010, or 2110 of any of the delivery devices (and/or body members) described herein. Since the clamp tube 450 extends from the rolling end portion 420 of the elongate body 405, the clamp tube 470 covers at least a portion of the resilient arms of delivery device 200*ᵢ₋ᵥᵢᵢ,* 2000*ᵢ₋ᵢᵢ.* Thus, the delivery device 200*ᵢ₋ᵥᵢᵢ,* 2000*ᵢ₋ᵢᵢ* is depicted in a restrained or closed configuration.

As further shown in Fig. 12D, in some embodiments, the clamp tube 450 and/or the second clamp tube end portion 470 may extend at least half the length, if not a majority of the length, of the delivery device 200*ᵢ₋ᵥᵢᵢ,* 2000*ᵢ₋ᵢᵢ* to transition the delivery device 200*ᵢ₋ᵥᵢᵢ,* 2000*ᵢ₋ᵢᵢ* from the natural expanded configuration to the restrained configuration. In some embodiments, as shown in Fig. 12D, in order to avoid the central stitch 60 extending from the delivery device 200*ᵢ₋ᵥᵢᵢ,* 2000*ᵢ₋ᵢᵢ,* the clamp tube 450 may extend up to the central stitch 60 (and/or central tie holes).

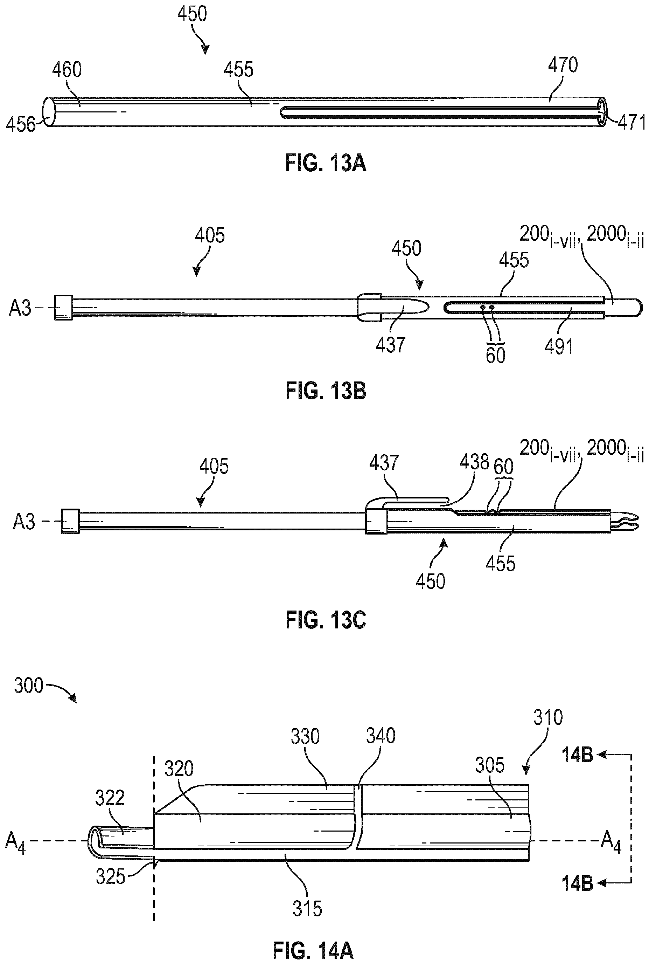

In some embodiments, as shown in Figs. 13A-13C, the clamp tube 450 may further include one or more longitudinal slots 471 defined in the tubular body 455 and particularly the second clamp tube end portion 470 of the tubular body 455. The longitudinal slot(s) 471 is configured to receive the central stitch 60 therein while the clamp tube 450 is slid distally over the delivery device 200*ᵢ₋ᵥᵢᵢ,* 2000*ᵢ₋ᵢᵢ.* In such embodiments, the clamp tube 450 may cover any length of the delivery device 200*ᵢ₋ᵥᵢᵢ,* 2000*ᵢ₋ᵢᵢ* without interfering with the central tie 60 extending therefrom.

In some embodiments, the longitudinal slot 471 and the finger clip 435 may be aligned along the length of the insertion member 400 (Figs. 13B-13C).

When attached, the insertion member 400, including the elongate body 405 and the clamp tube 450, and the delivery device 200*ᵢ₋ᵥᵢᵢ,* 2000*ᵢ₋ᵢᵢ* share a common central longitudinal axis A₃. The elongate body 405 may have a length that is longer than a typical trocar used for laparoscopic surgery.

The insertion member can be made of any suitable material. Some non-limiting examples of suitable materials include polyethylene, polypropylene, polyamides, polyaryl ether ketone (PAEK), acrylonitrile butadiene styrene (ABS), polyether ether ketone (PEEK), polyoxymethylene (POM), nitinol (NiTi), polyetherimide (PEI), polycarbonates (PC), and combinations thereof. In addition, the materials used to form the insertion member can be compatible with injection molding and/or extrusion manufacturing processes and compatible with standard sterilization methods, such as Ethylene Oxide and gamma radiation.

In some embodiments, the elongate body and the clamp tube may be manufactured separately using the same or different manufacturing processes and ultimately combined any time prior to use. For example, the elongate body may be made with injection molding processes while the clamp body may be made with extrusion processes, wherein the elongate body and the clamp tube are combined prior to packaging or after packaging and prior to use.

### V. Rolling Device

The rolling devices described herein are configured to prepare the implantable sheet and the delivery device for insertion into a patient. The rolling device is used to wrap the implantable sheet around an outer surface of the flexible delivery device to render the sheet in a rolled configuration. The rolling device may also be used by a surgeon to transfer the delivery device including the implantable sheet in a rolled configuration to a trocar for insertion into a patient. The rolling device, unlike the implantable sheet and delivery device is not intended to be inserted into a patient.

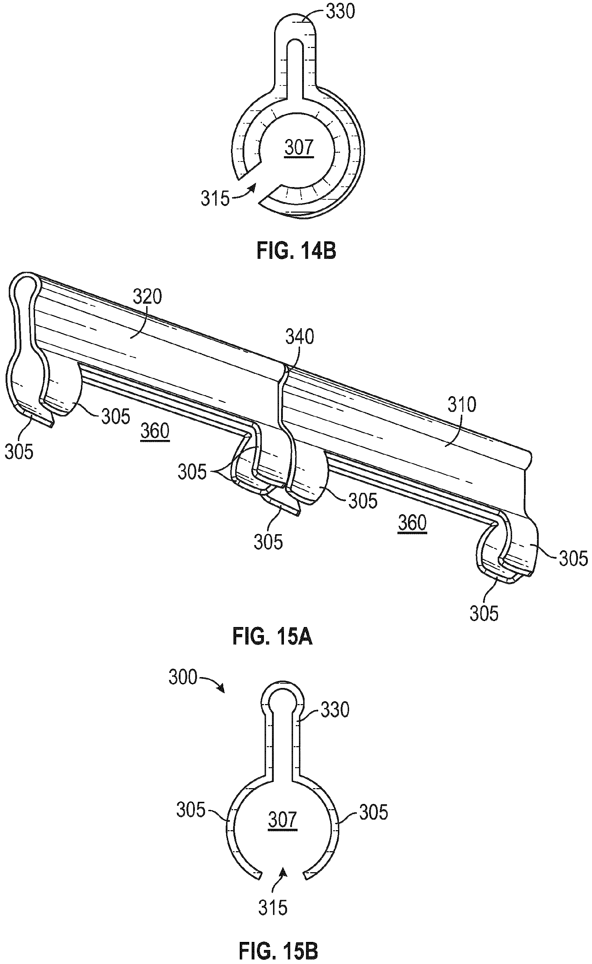

In Figs. 14A-14B, a rolling device 300 is shown including a generally tubular body 305 extending between a proximal end portion 310 and a distal end portion 320 of the rolling device 300, the tubular body 305 generally defines a channel 307 configured to receive the implantable sheet, the delivery device, and the clamp tube of the insertion member. The channel 307 extends the entire length of the tubular body 305 through both the proximal and distal end portions 310, 320 of the rolling device 300. A first slit 315 also extends the entire length of the tubular body creating an open generally tubular body 305 and/or an open channel 307. At least one fin 330 also extends along a length of the tubular body 305 and on an outer surface of the tubular body 305. The rolling devices described herein further include at least one tie handle opening 340 configured to receive the tie handle of the central tie prior to rolling. In some embodiments, the at least one tie handle opening 340 extends through one side of the fin 330 and tubular body 305 to the slit 315.

The first slit 315 is configured to allow passage of the implantable sheet, the delivery device, and the clamp tube of the insertion member into the channel 307 of the tubular body 305. The first slit 315 also provides the rolling device 300, which can be made of a rigid or semi-rigid material, the flexibility to expand or contract along the slit 315 as needed to accommodate different sizes of implantable sheet, delivery devices, and/or clamp tubes.

The rolling device 300 further includes a spout 322 and at least one flange 325 extending from the distal end portion 320. The spout 322 extends from the distal end portion 320 along the longitudinal axis A₄ of the device 300 and is configured to fit within or mate with a trocar opening to allow access into the trocar during insertion of the implantable sheet and delivery device. The at least one flange 325 is positioned on the distal end portion 320 proximal to the spout 322 and extends generally perpendicular to longitudinal axis A₄ of the device 300.

In some embodiments, as shown in Figs. 14A-14B, the fin 330 and the first slit 305 may not be vertically aligned with each other. In some embodiments, as shown in Figs. 15A-15B, the fin 330 and the first slit 305 may be vertically aligned, in particular vertically centered on, the rolling device 300.

In some embodiments, as shown in Fig. 15A, the rolling device 300 includes a fin 330 that extends the full length of the rolling device 300, while the tubular body 305 including the first slit 315 only extends across intermittent portions of the rolling device 300 creating open spaces 360 therebetween. As depicted, the tubular body 305 may be located on the end portions 310, 320 of the rolling device 300 and on either side of the tie handle opening 340. Also, in some embodiments, the rolling device may not include a spout or flange extending from a distal end portion thereof.

The fins as described herein are configured to be a handle for the surgeon to use when handling the rolling device. The fin cavities, of the fins described herein, participate in the global flexibility of the rolling device, to compress or relax the rolled implantable sheet and the delivery device inside the channel of the rolling device.

The rolling device can be made of any suitable material. Some non-limiting examples of suitable materials include polyethylene, polypropylene, polyamides, polyaryl ether ketone (PAEK), acrylonitrile butadiene styrene (ABS), polyether ether ketone (PEEK), polyoxymethylene (POM), nitinol (NiTi), polyetherimide (PEI), polycarbonates (PC), metals, and combinations thereof.

In addition to the various rolling devices described hereinabove, in some embodiments, the rolling device is a rolling device described in any of U.S Patent Nos. 8,317,808; 8734473; 9,364,311; 10,052,126; and 10,016,265, each of which are incorporated herein by reference.

In some embodiments, the implantable sheet is an implantable mesh and the rolling device is a mesh rolling device.

In some embodiments, the implantable sheet is an implantable mesh, the central tie is a suture, the delivery device is a mesh delivery device, the insertion member is mesh insertion member, and the rolling device is a mesh rolling device.

### VI. Methods of Use

The present disclosure also provides methods of treating or repairing soft tissue defects with the use of the various components of the kits described herein. The kits and components described herein are intended to be used in any variety of surgical procedures wherein a soft tissue defect needs repair. In some embodiments, the kits and components described herein may be used to repair various types of hernia including but not limited to hernia repair using an IPOM (i.e. intraperitoneal), TAPPS (i.e., preperitoneal), or TEPS (i.e., extraperitoneal) technique.

Any methods described herein directed to repairing a soft tissue defect or hernia is intended to be applicable specifically to ventral hernia repair and/or ventral hernia repair using an IPOM surgical technique.

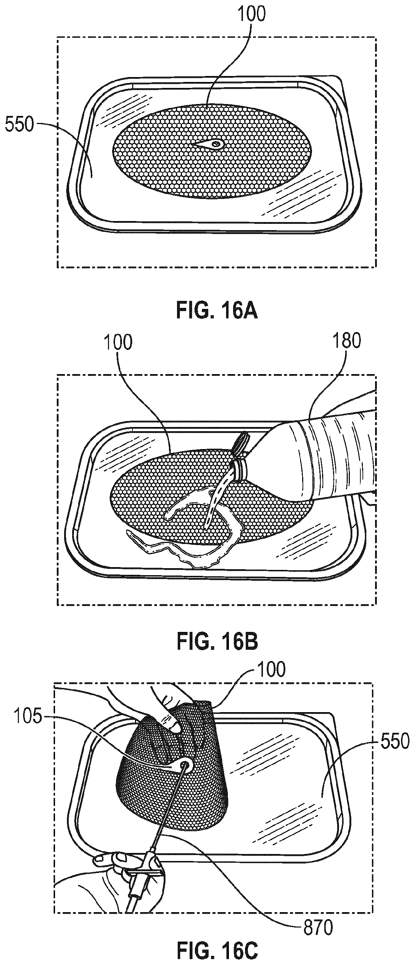

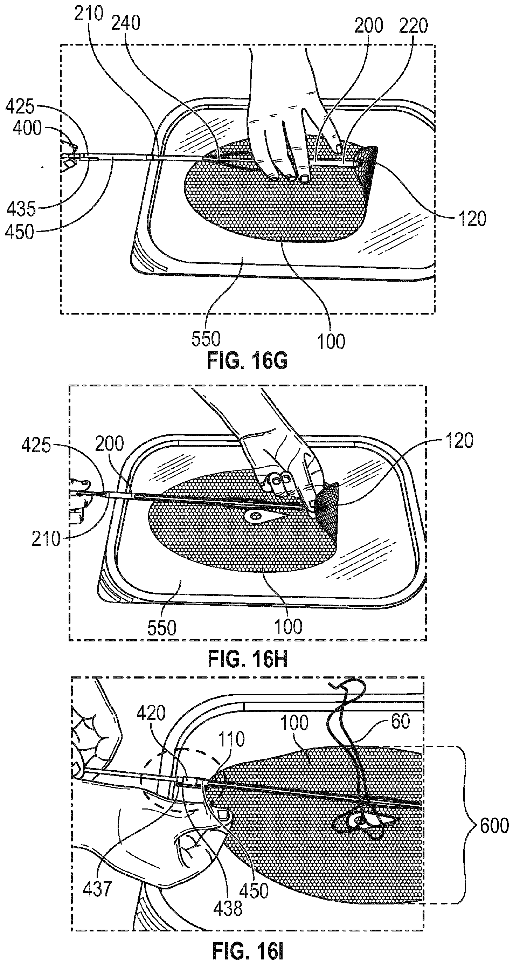

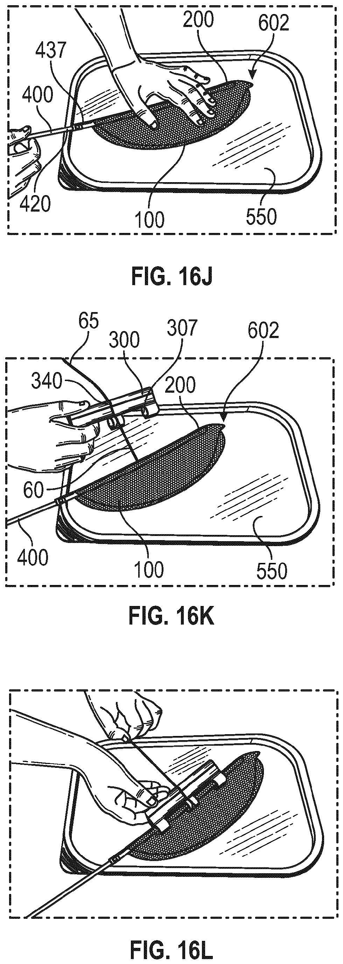

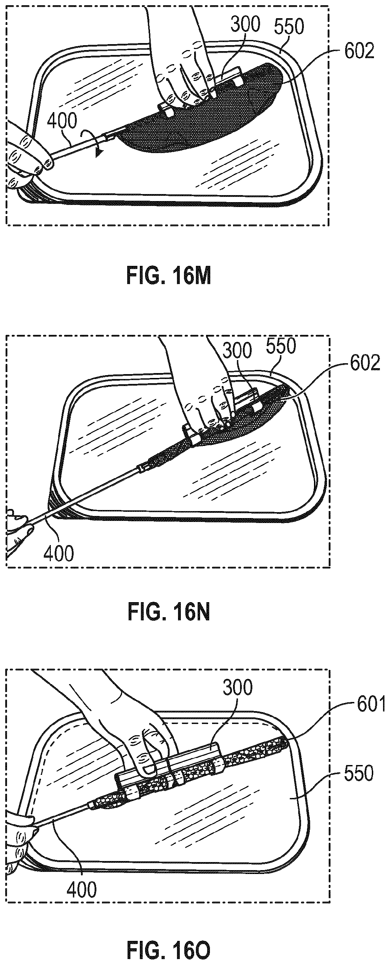

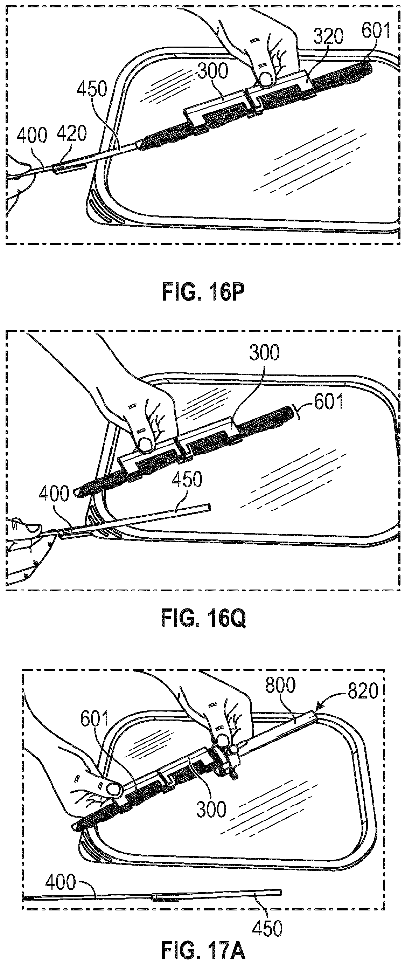

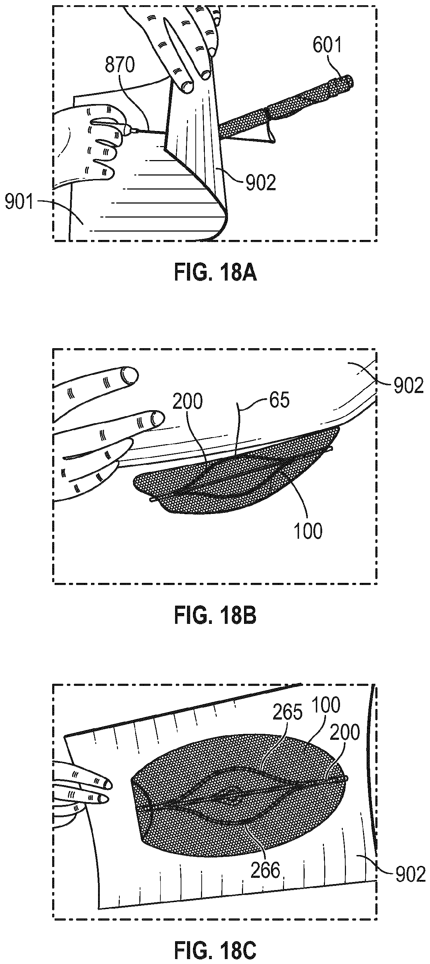

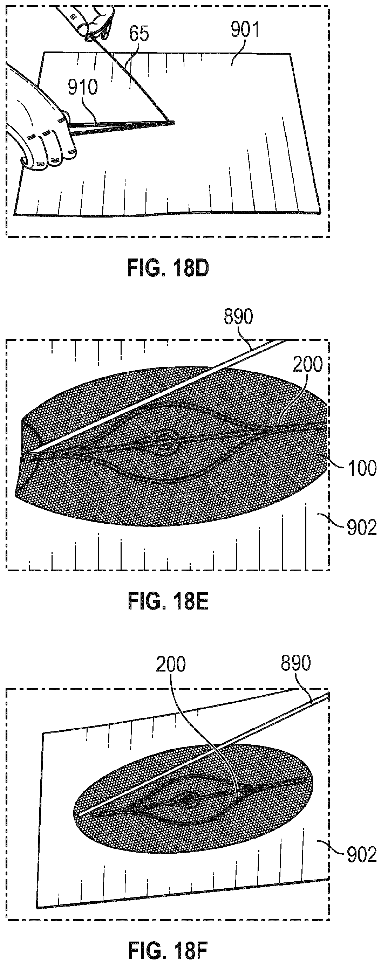

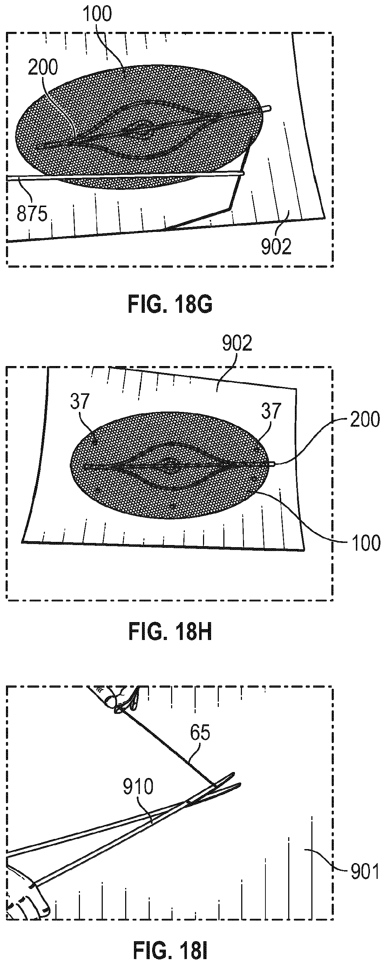

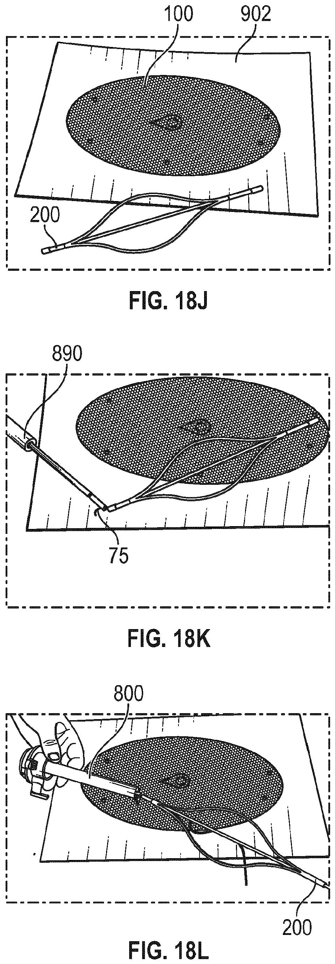

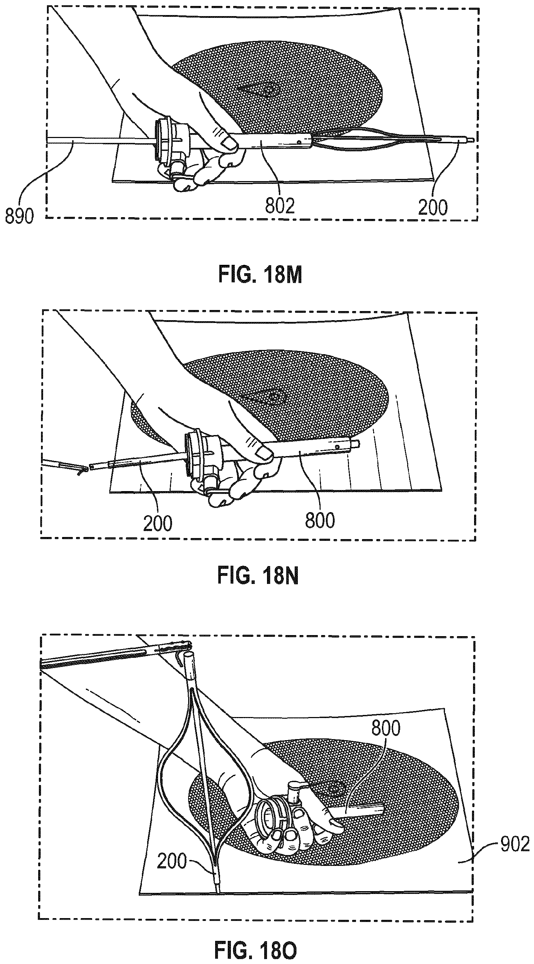

As provided in Figs. 16A-17G, in some embodiments, methods for repairing a soft tissue defect, such as a hernia or ventral hernia may include: preparing a fold sheet-delivery device assembly, preparing a rolled folded sheet-delivery device assembly, inserting the rolled folded sheet-delivery device assembly into the patient, orienting the implantable sheet, deploying the implantable sheet, fixating the implantable sheet, detaching the sheet from the delivery device and the central tie, and removing the central tie and the delivery device from the patient. The term delivery device throughout the present disclosure is intended to cover either or both of the one-piece or multi-piece delivery devices described herein. For clarity purposes, in Figs. 16A-17G (as well as Figs. 18A-18O), the delivery device will only be referenced as 200, i.e., any of the one-piece delivery devices 200*ᵢ₋ᵥᵢᵢ,* however the delivery device and any elements associated therewith are intended to cover the two-pieces delivery devices 2000*ᵢ₋ᵢᵢ* described herein as well.

As depicted in Figs. 16A-16B, in some embodiments, prior to preparing or forming a folded sheet-delivery device assembly, an implantable sheet 100, such as an implantable surgical mesh, may be wetted or hydrated with a sterile liquid 180. Some non-limiting examples of suitable sterile liquids 180 include water and/or saline. Wetting or hydrating the sheet 100 may increase the workability or flexibility of the sheet 100 prior to implantation. As depicted, in some embodiments, the implantable sheet 100 may be wetted or hydrated while maintained within the packaging 550, i.e., a blister pack, the sheet was stored in.

As depicted in Figs. 16C-16J a folded sheet-delivery device assembly 600 may be formed by: combining an implantable sheet 100 with a delivery device 200 as described herein such that a central tie 60 extends from the delivery device 200 through a central portion 105 of the sheet 100 and away from the sheet 100 a given length, the delivery device 200 being in the expanded configuration; securing a proximal and distal end portion 110, 120 of the implantable sheet 100 to a proximal and distal end portion of the delivery device 200 in a restrained configuration; and folding the implantable sheet 100 over the delivery device 200 to form a folded sheet.

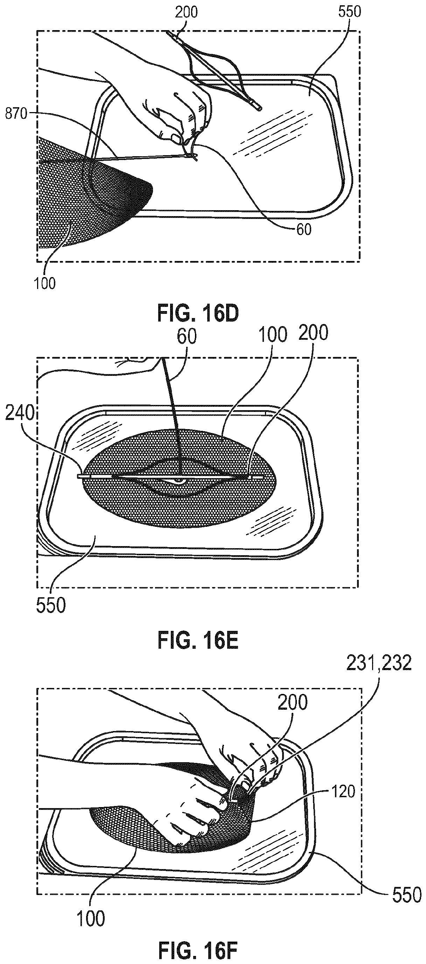

In some embodiments, the sheet 100 and the delivery device 200 may be combined outside of the patient by introducing a suture catcher 870, such as an Endoclose™, through a generally central portion 105 of the sheet 100 (Fig. 16C); grasping a portion of a central tie 60 extending from the one-piece or two-piece delivery device 200 as described herein (Fig. 16D); pulling the central tie 60 back through the central portion 105 of the sheet 100; and laying the sheet 100 generally flat with the delivery device 200 positioned thereon, the delivery device 200 in the expanded or open configuration (Fig. 16E). The given length of the central tie 60 extends away from the sheet 100 on a side of the sheet 100 opposite the delivery device 200.

In some embodiments, the sheet 100 may be secured to the delivery device 200 by: folding a distal end portion 120 of the implantable sheet 100 over one of an upper or lower jaw members 231, 232 of the delivery device 200 and secured within a slot defined within a distal end of the delivery device 200 (Fig. 16F); positioning or sliding the clamp tube 450 over the proximal end portion 210 of the delivery device 200 until the delivery device 200 transitions from the expanded configuration to the restrained configuration and/or the second socket 425 on the rolling end portion 420 of the insertion member 400 engages the proximal end portion 210 of the delivery device 200 (and/or the central arm 240) (Figs. 16G-16H); inserting a proximal end portion 110 of the implantable sheet 100 into the sheet gap 438 defined between the finger 437 and the distal end portion 420 (and/or the clamp tube 450) of the insertion member 400 (Fig. 16I); and folding the implantable sheet 100 into two, longitudinally, over the delivery device 200 forming a folded sheet-delivery device assembly 602 (Fig. 16J). The finger 437 secures and/or pinches the proximal end portion 110 of the sheet 100 to an exterior of the distal end portion 410 and/or the clamp tube 450 of the insertion member 400. Prior to folding, the proximal and distal end portions of the sheet are attached to the insertion member and the delivery device, respectively.

In some embodiments, the clamp tube may be positioned on the delivery device up to where the central tie extends therefrom (Fig. 12D). In some embodiments, the clamp tube may include at least one longitudinal tube slit. The at least one longitudinal tube slit configured to be aligned with the central tie extending from the delivery device thereby allowing the clamp tube to be positioned over a majority of the delivery device in the restrained configuration by avoiding the central tie (Figs. 13B-13C).

As depicted in Figs. 16K-16Q, the implantable sheet 100 of the folded sheet-delivery device assembly 602 can then be rolled to form a rolled folded sheet-delivery device assembly 601. The handle 65 of the central tie 60, extending from the folded sheet-delivery device assembly 602, can be positioned within a slit 340 extending generally perpendicular to a longitudinal channel 307 of the rolling device 300, where upon pulling of the handle 65 draws the folded sheet-delivery device assembly 602 into the channel 307 of the rolling device 300 (Figs. 16K-16L). The insertion member 400 may be rotated (see arrow in Fig. 16M) causing the folded sheet 100 to be rolled around the delivery device 200 in the restrained configuration and inside the channel 307 of the rolling device 300 to form a rolled folded sheet-delivery device assembly 601 (Figs. 16M-16O). The insertion member 400 is rotated until the center tie 60 and handle 65 are fully hidden inside the rolling device 300.

In some embodiments, as depicted in Figs. 16M-16O, prior to rolling of the sheet 100, the rolling device 300 may be held in a vertical relation to the blister pack 550 to press the distal end portion 220 of the delivery device 200 and sheet 100 against a corner of the blister pack 550. Then the sheet 100 may be rolled inside the rolling device 300 while being pressed against the blister pack 500 until the central tie 60 is fully received inside the rolling device 300.

In some embodiments, as depicted in Figs. 16P-16Q, after rolling of the sheet 100 and prior to insertion, the distal end portion 320 of the rolling device 300 may be pinched while the clamp tube 450 of the insertion member 400 is completely removed and/or slid away from the delivery device 200 and rolled sheet 100. The delivery device 200 remains in the restrained configuration with the sheet 100 and central tie 60 rolled thereover, after removal of the clamp tube 450.

After rolling, the rolled folded sheet-delivery device assembly 601 may be inserted into a patient. Prior to insertion, the proximal end portion 210 of the delivery device is connected to the inserting end portion 410 of the insertion member 400. In some embodiments, the first socket 415 of the inserting end portion 410 is configured to matingly engage and/or lock to the proximal end portion 210 of the delivery device 200.

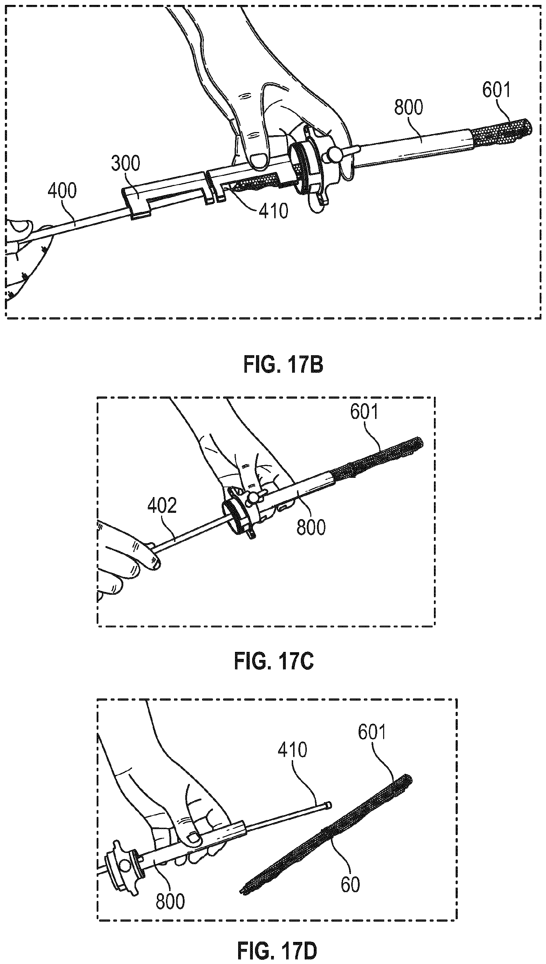

Once the sheet 100 is prepared in a rolled configuration and the rolled folded sheet-delivery device assembly 601 is prepared within the rolling device 300 and connected to the inserting end portion 410 of the insertion member 400, the rolled folded sheet-delivery device assembly 601 can be inserted into an abdominal cavity of a patient. For example, as shown in Figs. 17A-17D, in some embodiments, a method of inserting a rolled folded sheet-delivery device assembly 601 is described and includes the steps of: attaching a distal end portion 320 of the rolling device 300, such as the spout 322, to a trocar 800 extending from the patient's body, such as the abdomen; and moving or pushing the insertion member 400 in a distal direction through the channel 307 of the rolling device 300 and into the trocar 800 until the delivery device 200 including the implantable sheet 100 in a rolled configuration or the rolled folded sheet-delivery device assembly 601 completely enters a cavity within the patient, such as the abdominal cavity. In some embodiments, the insertion member 400 is further rotated while being pushed or slid distally into the trocar 800 and/or into the patient.

In some embodiments, the rolling device 300 and the insertion member 400 can be used as handles during the insertion process to carry the rolled folded sheet-delivery device assembly from the package to the trocar 800. A surgeon can easily grab the rolling device 300 on one end and the insertion member 400 on an opposite end, with the rolled folded sheet-delivery device assembly 601 positioned therebetween, to perform inserting the rolled folded sheet-delivery device assembly 601 into the patient. Since the rolling device 300 and the insertion member 400 are not intended to enter the patient, contact does not need to be avoided by the surgeon. This design also prevents or limits the amount of direct contact with the implantable sheet 100 and the delivery device 200 thereby reducing the likelihood of contamination.

Following insertion, as shown in Figs. 17A-17D, the rolled folded sheet-delivery device assembly 601 is free of both the rolling device 300 and the insertion member 400. However, the rolled folded sheet-delivery device assembly 601 may still be in a rolled configuration and needs to be deployed, oriented, and fixated within the cavity of the patient. For example, as shown in Figs. 18A-18D, in some embodiments, a method of deploying the rolled implantable sheet 100 or rolled folded sheet-delivery device assembly 601 is described. Figs. 18A-18O, are schematic images of the such methods, wherein the layer of material 900 is intended to represent a patient's soft tissue defect in need of repair, such as a hernia in an abdominal wall of a patient and the trocar 800 is schematically depicted.

As depicted in Figs. 18A-18D, the method of deploying includes introducing a suture catcher 870, such as an Endoclose™, through a center of the soft tissue defect from an outside 901 of the cavity; grasping the tie handle 65 of the central tie 60 extending from the rolled folded sheet-delivery device assembly 601 from an inside 902 of the cavity; pulling the tie handle 65 back through the soft tissue defect or hernia to the outside 901 of the cavity thereby releasing the rolled folded sheet 100 or rolled folded sheet-delivery device assembly 601 of constraint by the tie handle 65 on the inside 902 of the cavity; and securing the tie handle 65 on the outside 901 of the patient cavity. The tie handle 65 can be secured on the outside 901 of the cavity with any suitable surgical tool, such as a surgical clamp 910 or any suitable fastening means, such as a staple, tack, suture, clip, and the like. By initially passing the suture catcher 870 through the center of the defect, the placement of the sheet 100 when clamped is likely to also be centered on the defect.

As depicted in Figs. 18E-18F, since the delivery device 200 is still attached to the sheet 100 via the central tie 60, the pulling of the tie handle 65 out through the tissue defect or hernia, forces the delivery device 200 up against the underside of the soft tissue defect or hernia with the sheet 100 positioned therebetween. The combination of the central tie 60 and the delivery device 200 lifts and holds the sheet 100 up against the underside of the tissue defect. Since the tie handle 65 is no longer wrapped around the sheet 100 and the delivery device 200, the rolled mesh 100 begins to start to unroll and the one or more resilient arms 265, 266 will naturally expand outwardly returning to the expanded or open configuration forcing the sheet 100 to flatten against the inside 902 of the soft tissue defect.

During deployment or the method of deploying the implantable sheet, the distal portion 120 of the sheet 100 can be removed from the slot 230 of the delivery device 200. For example, a surgical grasper 890 can be used to grab the distal end portion 120 of the sheet 100 to free the sheet 100 of the slot 230.

Following deployment, final placement and/or positioning of the sheet can be determined and the sheet can be fixated in or around the tissue defect or hernia. For example, in some embodiments, as shown in Fig. 18G-18H, a method of placing and fixating the implantable sheet 100 is described and includes the steps of: manipulating the sheet 100 into a final position with a laparoscopic surgical grasper 890; and fixating the sheet 100 into tissue in or around the tissue defect 830 while the resilient arms 265, 266 of the delivery device 200 and the central tie 60 maintain the sheet 100 up against the inside 902 of the defect. In some embodiments, manipulation of the sheet may include and/or only require the rotation of the sheet because the central tie centers the sheet over the tissue defect or hernia. Any standard laparoscopic surgical grasper or standard surgical fastening device 875, such as a tack or clip applier, stapler, or suturing device, may be introduced into the patient or a cavity within a patient, such as the abdominal cavity, via one or more trocars. Because the sheet 100 remains suspended up against the defect while being positioned and/or fixated, both hands of the surgeon are free to work in unison to position and fixate the implantable sheet to the tissue. The sheet can be fixated using any suitable fixation means 37, including, but not intended to be limited to, sutures, clips, tacks, staples, adhesives, and the like.

Following fixation of the implantable sheet 100, the delivery device 200 and the central tie 60, including tie handle 65, can be separated from the sheet 100 and withdrawn from inside the patient's body cavity. For example, as shown in Figs. 18I-18O, in some embodiments, a method of withdrawing the delivery device 200 and central tie 60 from the site of implantation is described and includes the steps of: releasing the surgical clamp 910 on the outside 901 of the tissue defect thereby allowing the delivery device 200 in the expanded configuration (with or without the central tie 60 still attached to the delivery device 200) to fall to the bottom of the cavity; and withdrawing the delivery device 200 and/or central tie 60 from inside the cavity of the patient.

In some embodiments, to reduce and/or avoid contamination to the cavity, rather than releasing the surgical clamp 910, the surgical clamp 910 may remain clamped to a first external portion of the central tie 60, i.e., a first of the two (or more) threads extending externally from the patient, while a second external portion of the central tie 60, i.e., a second of the two (or more) threads extending externally from the patient, may be cut. The second external portion ultimately freed of the delivery device and withdrawn from the patient by the pulling of the clamp 910 away from the patient's body, thereby allowing only the delivery device to fall to the bottom of the cavity inside the patient.

In some embodiments, the laparoscopic surgical grasper 890 can be utilized to grab the distal end of the delivery device 200, and particularly a looped suture 75 positioned through the suture aperture 222 on the distal end portion 220 of the delivery device 200, to slide the distal end portion 220 of delivery device 200 back through the trocar 800 and out of the patient. The walls of the trocar 800 will force the one or more resilient arms 265, 266 to retract as the delivery device 200 is pulled out through the trocar 800. Because the delivery device 200 is flexible, the delivery device 200 does not need to be perfectly aligned with the trocar 800 to be removed.

In some embodiments, a method of preparing a rolled folded sheet-delivery device assembly includes: combining an implantable sheet, a central stitch, and a one-piece or multiple-piece delivery device to form a sheet-delivery device assembly, the delivery device including one or more resilient arms in an expanded configuration, positioning a clamp tube of an insertion member over at least a portion of the delivery device transitioning the one or more resilient arms into a restrained configuration, folding the implantable sheet longitudinally over the delivery device and the clamp tube to form a folded sheet-delivery device assembly, and rolling the folded sheet of the folded sheet-delivery device assembly around the delivery device within a rolling device to form a rolled folded sheet-delivery device assembly.

In some embodiments, a method of treating a soft tissue defect includes: inserting a rolled folded sheet-delivery device assembly into a cavity of a patient via a trocar, the assembly including an implantable sheet, a one-piece or multiple-piece delivery device, and a central tie extending from the one-piece or two-piece delivery device, deploying the implantable sheet inside the cavity beneath the tissue defect, fixating the implantable sheet to tissue surrounding the defect on the inside of the cavity, and withdrawing the delivery device from the patient.

In some embodiments, the method of treating a soft tissue defect may further include: introducing a suture catcher from an outside of the cavity through a center of the tissue defect to an inside of the cavity to grab the central tie, pulling the suture catcher with the central tie back through tissue defect to the outside of the cavity until the sheet and the delivery device are pressed up against the tissue defect inside the cavity, securing the central tie with a surgical clamp on the outside of the cavity, and freeing the proximal and distal end portions of the implantable sheet from the delivery device and the insertion member.

Each of the components and/or kits described herein may be stored in any package suitable for maintaining the components and/or kits under sterile conditions. Some non-limiting examples includes peelable packaging, foil packaging, Tyvek packaging, plastic molded packaging, and the like.

It will be understood that various modifications may be made to the embodiments disclosed herein. Thus, those skilled in the art will envision other modifications within the scope and spirit of the disclosure.

The invention may be described by reference to the following numbered paragraphs:-
1. A two-piece delivery device configured to deliver an implantable sheet, the delivery device comprising:
   a first body member including a central arm extending between a first proximal end portion and a first distal end portion, the first proximal end portion including a tube defining a tube lumen and the first distal end portion including a body locking recess defined therein, and
   a second body me member including a pair of resilient arms extending between a second proximal end portion and a second distal end portion, the second proximal end portion including a handle received within the tube lumen of the first body member, and the distal end portion including a locking member received within the body locking recess of the first body member locking the first body member to the second body member.
2. The two-piece delivery device of paragraph 1, wherein the tube further comprises a tube slot extending a length of the tube forming a split tube.
3. The two-piece delivery device of paragraph 1, wherein the central arm comprises at least a first area which defines a T-shaped cross-section.
4. The two-piece delivery device of paragraph 3, wherein the central arm further comprises a second area which defines a T-shaped cross-section, the second area separated longitudinally along the central arm from the first area by a space.
5. The two-piece delivery device of paragraph 4, wherein the central arm further comprises first and second tie holes defined therethrough and positioned on the space between the first and second areas.
6. The two-piece delivery device of paragraph 1, wherein the first distal end portion of the first body member further comprises a slot distal the locking recess, the slot separating the first distal end portion of the first body member into an upper and lower jaw member, the slot configured to secure a distal end portion of an implantable sheet between the upper and lower jaw members.
7. The two-piece delivery device of paragraph 1, wherein the pair of resilient arms are configured to transition between an expanded configuration with the pair of resilient arms spaced farthest apart from each other and a restrained configuration with the pair of resilient arms generally adjacent each other.
8. The two-piece delivery device of paragraph 1, wherein the handle is configured to slide longitudinally within the tube lumen of the tube.
9. A two-piece delivery device configured to deliver an implantable sheet, the delivery device comprising:
   a first body member including a pair of resilient arms extending between a first proximal end portion and a first distal end portion, the first proximal end portion including a first tube defining a first tube lumen and the first distal end portion including a second tube defining a second tube lumen, the second tube further including a body locking recess defined therein, and
   a second body member including an arm extending between a second proximal end portion and a second distal end portion, the second proximal end portion received within the first tube lumen of the first body member, and the second distal end portion including a body locking member and a slot distal the locking member, the locking member received within the body locking recess of the first body member locking the first body member to the second body member and the slot separating the second distal end portion of the second body member into an upper and lower jaw member, the slot configured to secure a distal end portion of an implantable sheet between the upper and lower jaw members.
10. The two-piece delivery device of paragraph 9, wherein the first tube lumen defines a generally square-shaped cross-section.
11. The two-piece delivery device of paragraph 9, wherein the first tube lumen further comprises a guide member extending a length of the first tube lumen and protruding into at least one side of the square -shaped cross-section.
12. The two-piece delivery device of paragraph 11, wherein at least the second proximal end portion of the second body member defines a generally square-shaped cross-section including at least one groove extending a length of the second body member, the groove configured to accommodate the guide member.
13. The two-piece delivery device of paragraph 9, wherein each of the pair of the resilient arms define a half-moon cross-section.
14. The two-piece delivery device of paragraph 9, wherein the central arm further comprises first and second tie holes defined therethrough.
15. The two-piece delivery device of paragraph 9, wherein the pair of resilient arms are configured to transition between an expanded configuration with the pair of resilient arms spaced farthest apart from each other and a restrained configuration with the pair of resilient arms generally adjacent each other.
16. The two-piece delivery device of paragraph 9, wherein the first tube is configured to slide longitudinally along the second proximal end portion of the second body member.
17. A one-piece delivery device configured to deliver an implantable sheet, the delivery device comprising:
   a body member extending from a proximal end portion to a distal end portion, the body member including a plurality of pairs of resilient arms including at least a first and second pair of resilient arms, the first pair of resilient arms affixed to the proximal end portion of the body member, the second pair of resilient arms affixed to the distal end portion of the body member, and a central arm positioned at least between the first and second pairs of resilient arms, wherein at least one of the first or second pair of resilient arms are configured to transition between an expanded configuration and a restrained configuration.
18. The one-piece delivery device of paragraph 17, wherein the central arm further extends from: the proximal end portion of the body member to a distal end portion of the first pair of resilient arms, the proximal end portion of the second pair of resilient arms to the distal end portion of the body member, or both, without preventing the first and second resilient arms from transitioning between the expanded configuration and the restrained configuration.
19. A one-piece delivery device configured to deliver an implantable sheet, the delivery device comprising:
   a body member extending from a proximal end portion to a distal end portion, the body member including a pair of resilient arms and a central arm positioned between the proximal and distal end portions thereof, at least one of the pair of resilient arms or the central arm being affixed to both the proximal and distal end portions of the body, wherein the body is configured to transition between an expanded configuration with the pair of resilient arms spaced farthest apart from each other and a restrained configuration with the pair of resilient arms generally adjacent each other.
20. The one-piece delivery device of paragraph 1, wherein the central arm is affixed to both the proximal and distal end portions of the body member.
21. The one-piece delivery device of paragraph 20, wherein the pair of resilient arms are affixed to both the proximal and distal end portions of the body member.
22. The one-piece delivery device of paragraph 21, wherein the central arm is a resilient arm spaced from the pair of resilient arms and the central arm includes a generally S-shaped central portion in the expanded configuration and is generally adjacent the pair of resilient arms in the restrained configuration.
23. The one-piece delivery device of paragraph 19, wherein the distal end portion of the body member further comprises a slot separating the distal end portion of the body member into an upper and lower jaw member, the slot configured to secure a distal end portion of an implantable sheet between the upper and lower jaw members.
24. The one-piece delivery device of paragraph 20, wherein proximal end portion of the body member further comprises a tube configured to receive and maintain a proximal end portion of each of the pair of resilient arms therein.
25. The one-piece delivery device of paragraph 24, wherein a distal end portion of each of the pair of resilient arms is affixed to the distal end portion of the body member and a proximal end portion of each of the pair of resilient arms are configured to be received and maintained in the tube.
26. The one-piece delivery device of paragraph 21, wherein the central arm is flat rod.
27. The one-piece delivery device of paragraph 21, wherein the central arm comprises a first arm part and a second arm part space longitudinally from the first arm part, wherein the first arm part is affixed to the proximal end portion of the body member, the second arm part is affixed to the distal end portion of the body member, and a central gap is positioned between the first and second arm parts of the central arm.
28. The one-piece delivery device of paragraph 27, wherein the central arm further comprises first and second tie holes, the first tie hole defined through a distal end portion of the first arm part and the second tie hole defined through a proximal end portion of the second arm part.
29. The one-piece delivery device of paragraph 27, wherein the pair of resilient arms form one of an elliptical shape or an eye-ball shape.
30. The one-piece delivery device of paragraph 20, wherein the pair of resilient arms are affixed to the proximal end portion of the body member and free of the distal end portion of the body member.
31. A surgical kit comprising:
   a one-piece or multi-piece delivery device configured to deliver an implantable sheet; and
   an insertion member including:
      an insertion member having an elongate body extending between an insertion end portion including a first socket and a rolling end portion including a second socket, the insertion member defining a first longitudinal axis, and each of the first and second sockets being configured to matingly engage the proximal end portion of the one-piece delivery device,
      a clamp tube having a tubular body extending between a first clamp tube end portion configured to attach to and extend away from the rolling end portion of the insertion member and a second clamp tube end portion opposite the first tube end portion, the second clamp tube end portion free of the insertion member and configured to receive at least the proximal end portion of the delivery device therein to transition a pair of resilient arms from an expanded configuration to a restrained configuration, and
      a finger extending between a fixed first end portion and a free second end portion, the fixed first end portion affixed to the rolling end portion of the insertion member, the free second end portion extending away from the rolling end portion of the insertion member and over at least the first clamp tube end portion of the clamp tube, the free second end portion of the finger defining a sheet gap between the finger and the clamp tube, the sheet gap configured to receive and maintain a proximal end of an implantable mesh.
32. The surgical kit of paragraph 31, wherein the clamp tube further comprises one or more longitudinal slots defined therethrough.
33. A method of preparing a rolled folded sheet-delivery device assembly comprising:
   combining an implantable sheet, a central stitch, and a delivery device to form a sheet-delivery device assembly, the delivery device including one or more resilient arms in an expanded configuration,
   positioning a clamp tube of an insertion member over at least a portion of the delivery device transitioning the one or more resilient arms into a restrained configuration,
   folding the implantable sheet longitudinally over the delivery device and the clamp tube to form a folded sheet-delivery device assembly,
   rolling the folded sheet of the folded sheet-delivery device assembly around the delivery device within a rolling device to form a rolled folded sheet-delivery device assembly.
34. A method of treating a soft tissue defect, the method comprising:
   inserting a rolled folded sheet-delivery device assembly into a cavity of a patient via a trocar, the assembly including an implantable sheet, a delivery device, and a central tie extending from the delivery device,
   deploying the implantable sheet inside the cavity centering the implantable sheet beneath the tissue defect,
   fixating the implantable sheet to tissue surrounding the defect on the inside of the cavity, and
   withdrawing the delivery device from the patient.
35. The method of treating a soft tissue defect of paragraph 34, wherein deploying the implantable sheet further comprises:
   introducing a suture catcher from an outside of the cavity through a center of the tissue defect to an inside of the cavity to grab the central tie,
   pulling the suture catcher with the central tie back through tissue defect to the outside of the cavity until the sheet and the delivery device are pressed up against the tissue defect inside the cavity,
   securing the central tie with a surgical clamp on the outside of the cavity, and
   freeing the implantable sheet from the delivery device.

## Claims

1. A two-piece delivery device configured to deliver an implantable sheet, the delivery device comprising:
a first body member including a central arm extending between a first proximal end portion and a first distal end portion, the first proximal end portion including a tube defining a tube lumen and the first distal end portion including a body locking recess defined therein, and
a second body me member including a pair of resilient arms extending between a second proximal end portion and a second distal end portion, the second proximal end portion including a handle received within the tube lumen of the first body member, and the distal end portion including a locking member received within the body locking recess of the first body member locking the first body member to the second body member.

2. The two-piece delivery device of claim 1, wherein the tube further comprises a tube slot extending a length of the tube forming a split tube.

3. The two-piece delivery device of any preceding claim, wherein the central arm comprises at least a first area which defines a T-shaped cross-section,
preferably wherein the central arm further comprises a second area which defines a T-shaped cross-section, the second area separated longitudinally along the central arm from the first area by a space, preferably wherein the central arm further comprises first and second tie holes defined therethrough and positioned on the space between the first and second areas.

4. The two-piece delivery device of any preceding claim, wherein the first distal end portion of the first body member further comprises a slot distal the locking recess, the slot separating the first distal end portion of the first body member into an upper and lower jaw member, the slot configured to secure a distal end portion of an implantable sheet between the upper and lower jaw members.

5. The two-piece delivery device of any preceding claim, wherein the pair of resilient arms are configured to transition between an expanded configuration with the pair of resilient arms spaced farthest apart from each other and a restrained configuration with the pair of resilient arms generally adjacent each other.

6. The two-piece delivery device of any preceding claim, wherein the handle is configured to slide longitudinally within the tube lumen of the tube.

7. A two-piece delivery device configured to deliver an implantable sheet, the delivery device comprising:
a first body member including a pair of resilient arms extending between a first proximal end portion and a first distal end portion, the first proximal end portion including a first tube defining a first tube lumen and the first distal end portion including a second tube defining a second tube lumen, the second tube further including a body locking recess defined therein, and
a second body member including an arm extending between a second proximal end portion and a second distal end portion, the second proximal end portion received within the first tube lumen of the first body member, and the second distal end portion including a body locking member and a slot distal the locking member, the locking member received within the body locking recess of the first body member locking the first body member to the second body member and the slot separating the second distal end portion of the second body member into an upper and lower jaw member, the slot configured to secure a distal end portion of an implantable sheet between the upper and lower jaw members.

8. The two-piece delivery device of claim 7, wherein the first tube lumen defines a generally square-shaped cross-section.

9. The two-piece delivery device of claim 7, wherein the first tube lumen further comprises a guide member extending a length of the first tube lumen and protruding into at least one side of the square -shaped cross-section, preferably wherein at least the second proximal end portion of the second body member defines a generally square-shaped cross-section including at least one groove extending a length of the second body member, the groove configured to accommodate the guide member.

10. The two-piece delivery device of any of claims 7-9, wherein each of the pair of the resilient arms define a half-moon cross-section.

11. The two-piece delivery device of any of claims 7-10, wherein the central arm further comprises first and second tie holes defined therethrough.

12. The two-piece delivery device of any of claims 7-11, wherein the pair of resilient arms are configured to transition between an expanded configuration with the pair of resilient arms spaced farthest apart from each other and a restrained configuration with the pair of resilient arms generally adjacent each other.

13. The two-piece delivery device of any of claims 7-12, wherein the first tube is configured to slide longitudinally along the second proximal end portion of the second body member.

14. A surgical kit comprising:
a two-piece delivery device configured to deliver an implantable sheet according to any preceding claim; and
an insertion member including:
an insertion member having an elongate body extending between an insertion end portion including a first socket and a rolling end portion including a second socket, the insertion member defining a first longitudinal axis, and each of the first and second sockets being configured to matingly engage the proximal end portion of the one-piece delivery device,
a clamp tube having a tubular body extending between a first clamp tube end portion configured to attach to and extend away from the rolling end portion of the insertion member and a second clamp tube end portion opposite the first tube end portion, the second clamp tube end portion free of the insertion member and configured to receive at least the proximal end portion of the delivery device therein to transition a pair of resilient arms from an expanded configuration to a restrained configuration, and
a finger extending between a fixed first end portion and a free second end portion, the fixed first end portion affixed to the rolling end portion of the insertion member, the free second end portion extending away from the rolling end portion of the insertion member and over at least the first clamp tube end portion of the clamp tube, the free second end portion of the finger defining a sheet gap between the finger and the clamp tube, the sheet gap configured to receive and maintain a proximal end of an implantable mesh.

15. The surgical kit of claim 14, wherein the clamp tube further comprises one or more longitudinal slots defined therethrough.
